# EUROPEAN PATENT APPLICATION

(11) **EP 1 953 140 A1**
(43) Date of publication of application: **06.08.2008**
(21) Application number: 07001537.5
(22) Date of filing: 24.01.2007
(51) Int. Cl.: C07D 205/08, A61K 31/397, A61P 3/00

(54) **Process for the preparation of ezetimibe and derivatives thereof**

(71) Applicant: KRKA, 8501 Novo mesto (SI)
(72) Inventor: Stimac, Anton, 1000 Ljubljana (SI); Mohar, Barbara, SI-1290 Grosuplje (SI); Stephan, Michel, 92170 Vanves (FR); Bevc, Mojca, 8233 Mirna (SI); Zupet, Rok, 1000 Ljubljana (SI); Gartner, Andrej, 1000 Ljubljana (SI); Kroselj, Vesna, 8310 Sentjernej (SI); Smrkolj, Matej, 1411 Izlake (SI)
(74) Representative: Becker Kurig Straus

(57) **Abstract**

The present invention relates to the method of preparing of ezetimibe and in particular to novel intermediates for its synthesis and an improved process for preparing such intermediates. Said intermediates may be obtained in high yields and purity in a fast and cost efficient manner. The present invention relates to a novel crystalline form of ezetimibe as well.

## Description

The present invention relates to an improved process for the preparation of 1-(4-fluorophenyl)-3(R)-[3-(4-fluorophenyl)-3(S)-hydroxypropyl]-4(S)-(4-hydroxyphenyl)-2-azetidinone, based on [ruthenium-R³R⁴NSO₂-1,2-diamine] catalyzed asymmetric transfer hydrogenation of p-fluoroacetophenones. Said intermediates may be obtained in high yields and purity in a fast and cost efficient manner.

Hypercholesterolemia and high blood- or plasma-cholesterol are common diseases in the well-situated countries. Hypercholesterolemia has been implicated in atherosclerosis, hardening-of-arteries, heart-attack, and is one of several conditions that may lead to coronary and artery diseases. The risk group includes the overweight, smokers, those with a poor diet (e.g. one rich in saturated fats), those who take inadequate exercise and suffering from stress. For such risk individuals, as well as those tested and found to have unduly high plasma cholesterol levels, a variety of treatments have been proposed, e.g. changes in diet and habits, increased exercise, etc. However such treatments are not always easy to enforce and therefore there exists a constant need for medicinal treatments which have been effective at reducing plasma cholesterol levels.

Statins (e.g. fluvastatin, simvastatin, lovastatin, atorvastatin, rosuvastatin), and in particular simvastatin, are commonly used in the treatment or prevention of high cholesterol level in individuals. Also other compounds having a different mode of action with regard to a reduction of blood cholesterol levels have been proposed for use. Among them is a well known drug ezetimibe, a class of lipid-lowering compounds that selectively inhibits the intestinal absorption of cholesterol and related phytosterols.

Ezetimibe with chemical name 1-(4-fluorophenyl)-3(R)-[3-(4-fluorophenyl)-3(S)-hydroxypropyl]-4(S)-(4-hydroxyphenyl)-2-azetidinone and identified by the structure formula (Ia) was disclosed in EP 0720599.

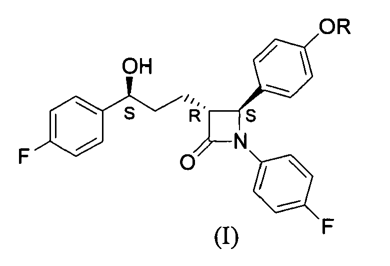

### Ezetimibe structural formula Ia : R = H

The mechanism of absorption and resorption inhibition of cholesterol of ezetimibe involves increased excretions of cholesterol and its intestinally generated metabolites with the faeces. This effect results in lowered body cholesterol levels, increased cholesterol synthesis, and decreased triglyceride synthesis. The increased cholesterol synthesis initially provides for the maintenance of cholesterol levels in the circulation, levels that eventually decline as the inhibition of cholesterol absorption and resorption continues. The overall effect of the drug action is the lowering of cholesterol level in the circulation and tissues of the body. Ezetimibe is also suspected to reduce plasma concentrations of the noncholesterol sterols sitosterol and campesterol, suggesting an effect on the absorption of these compounds as well.

Different synthetic routes to ezetimibe and derivatives thereof have been described in literature wherein the key-step relies on the asymmetric reduction of α-functionalized p-fluoroacetophenone intermediates with the general formula (IIa), (IIb), (IIIb) or (IV).

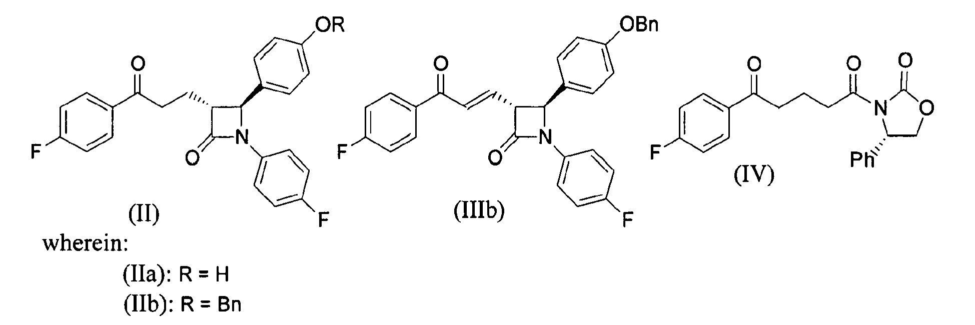

In EP 0 906 278 and EP 0 720 599, ezetimibe (Ia) and its derivative (Ib) (formula (I) wherein R = Bn) were prepared through borane reduction of the corresponding ketone (IIa) and (IIb). The reduction was catalyzed by 10 mol% of (R)-tetrahydro-1-methyl-3,3-diphenyl-1H,3H-pyrrolo-[1,2-c][1,3,2]oxazaborolidine at -20 °C followed by O-debenzylation for (Ib). The same type of reduction was applied to ketones (IIIb) and (IV) (disclosed in EP 0 707 567). According to this literature, the alcohols obtained from reduction of (IIa), (IIb) or (IV) were isolated in 70 to 80% yield with a diastereomeric ratio (dr) of 96:4 to 99:1. The reduction of compound with the general formula (IIIb) led to a dr of 88:12. These processes generate a stoichoimetric amount of borate waste salts.

An alternative synthesis of ezetimibe, a microbial reduction with high dilution of ketone (IIa) to ezetimibe as a single diastereomer was described in EP 1 169 468. However, this process is low yielding (15% yield).

Furthermore, the intermediate ketone (IIb) used in the above mentioned literature is syrupy and it can only be obtained pure after a tedious chromatographic purification.

On the another hand, alternative means of stereoselective reduction of arylketones excluding the stoichiometric use of borane reagents have been disclosed. 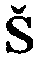 terk et al. (Tetrahedron: Asymmetry 2002, 13, 2605-2608) performed the efficient asymmetric transfer hydrogenation of various classes of ketones using formic acid/triethylamine mixture catalyzed by optically pure ruthenium or rhodium complexes of N-(N,N-dialkylsulfamoyl)-1,2-diamine ligands. The catalysts can be prepared in situ and do not require any special inert gas manipulation.

Polymorphic forms of ezetimibe are described for example in WO 2005/009955, WO 2005/062897, WO 2006/060808, US 2006/0234996, IPCOM000131677D disclosing mainly anhydrous and hydrous crystalline forms, different mixtures thereof and amorphous form of ezetimibe. The obtained polymorphic form depends on the solvent used in the recrystallization step and on water content of the final product (WO 2006/060808). Ezetimibe form A, form B and the process for the preparation thereof is disclosed in WO 2006/060808. The solvated forms of ezetimibe form B are disclosed in IPCOM000131677D.

Accordingly, there is a need in the art to provide an alternative synthesis of ezetimibe permitting the provision of higher yields of said compound with higher purity and obtained in a cost intensive overall synthesis.

The above mentioned problem has been solved by providing novel intermediates of ezetimibe and a modified reaction scheme allowing the provision of said intermediates as well as the end product in higher optical purity and higher yield.

### Summary of the Invention

The present invention relates to an efficient process of synthesis of ezetimibe (Ia) (formula (I) wherein R = H) based on a metal-catalyzed asymmetric transfer hydrogenation step of p-fluoroacetophenones of general formula (II) (wherein R is as defined below ) to their corresponding alcohols of general formula (I) (wherein R is as defined above). The metal catalysts are based on ruthenium complexes of optically pure N-sulfamoyl-1,2-diamine ligands (R³R⁴NSO₂-1,2-diamine wherein R³ and R⁴ are as defined below).

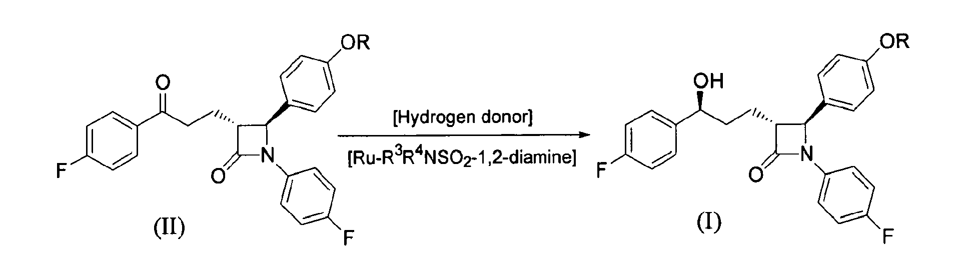

Such technology allows an increased productivity as the product alcohol (I) is easily isolated with high optical purity and in high yield, and does not generate cumbersome salts.

Another embodiment of the present invention relates to an improved synthesis of the p-fluoroacetophenone intermediates of general formula (II) through novel intermediates of general formula (V), wherein Z is COC1, COOH, CO₂Me, CON(Me)OMe, or CON(Me)OEt and R is a mono or a disubstituted benzyl or a silyl group.

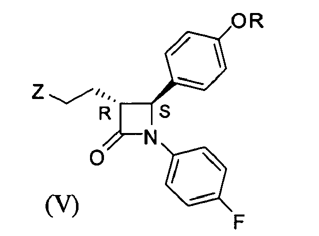

According to the improved synthesis, intermediates of general formula (V) can lead to ketones of general formula (II) (wherein R is as defined below) as crystalline compounds which are easily purified on an industrial scale than the syrupy ketone of general formula (IIb) known from the state of the art.

According to still another embodiment, the present invention relates to novel intermediates of general formula (V) wherein Z and R are as defined above.

The synthetic route for the preparation of ezetimibe and derivatives thereof according to the present invention is presented in Schemes 1 and 2.

In another embodiment, the present invention provides novel crystalline form S ezetimibe and the process for its preparation.

In another embodiment the present invention provides a pharmaceutical composition containing ezetimibe prepared according to the process of the present invention

### Brief Description of the Figures

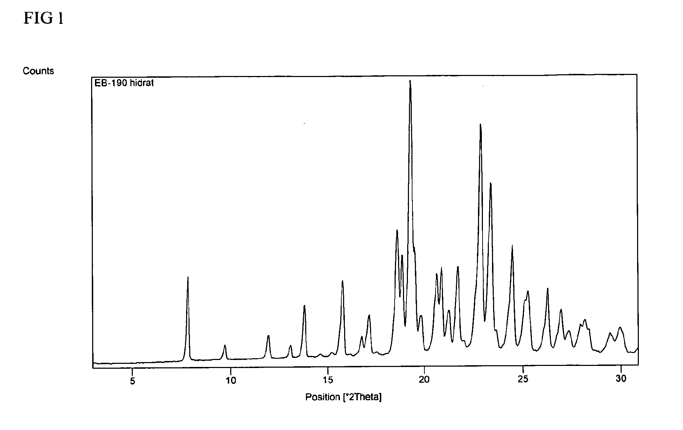

Figure 1: Powder X-ray diffraction pattern of hydrated form H
Figure 2: Powder X-ray diffraction pattern of anhydrous form A
Figure 3: Powder X-ray diffraction pattern of form S

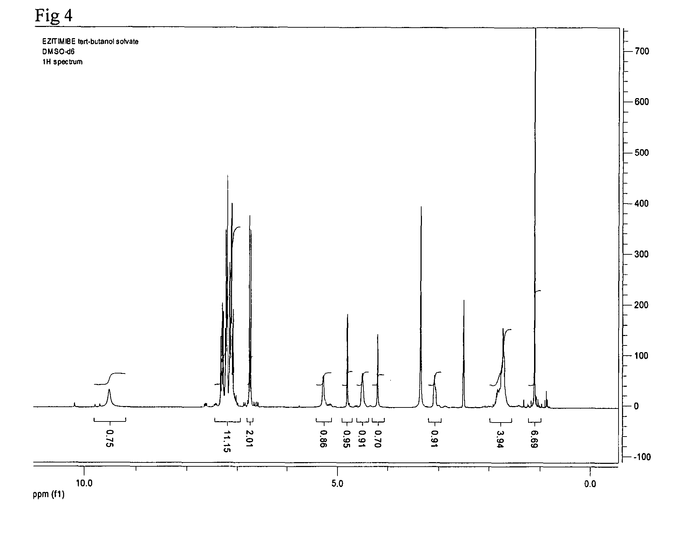

Figure 4: NMR spectra of form S

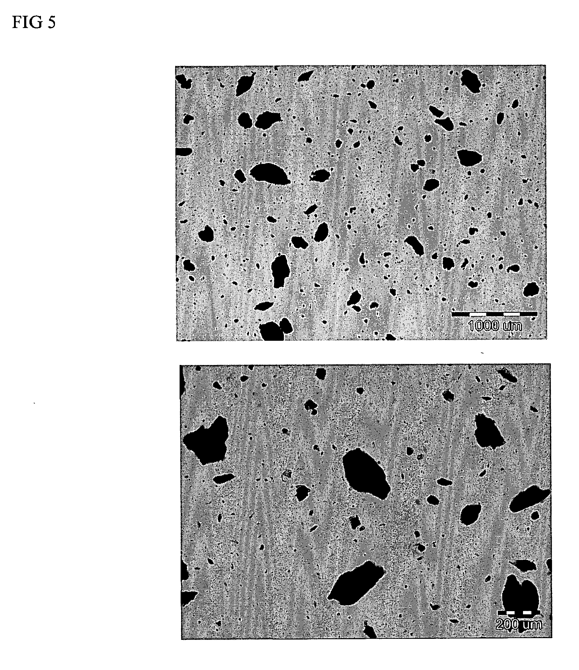

Figure 5: Form S as seen through a microscope with different magnitudes
Figure 6: Powder X-ray diffraction pattern of Methyl 3-[(2S,3R)-1-(4-fluorophenyl)-2-(4-hydroxyphenyl)-4-oxoazetidinyl]propionate
Figure 7: Powder X-ray diffraction pattern of Methyl 3-{(3R,4S)-1-(4-fluorophenyl)-2-oxo-4-[4-(trityloxy)phenyl]azetidin-3-yl}propionate
Figure 8: Powder X-ray diffraction pattern of Methyl 3-{(2S,3R)-2-[4-(benzyloxy)phenyl]-1-(4-fluorophenyl)-4-oxoazetidin-3-yl }propionate

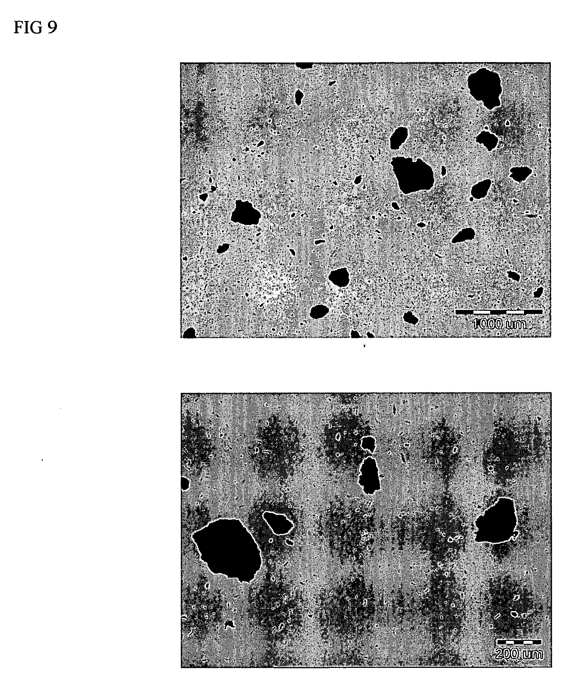

Figure 9: Anhydro form A as seen through a microscope

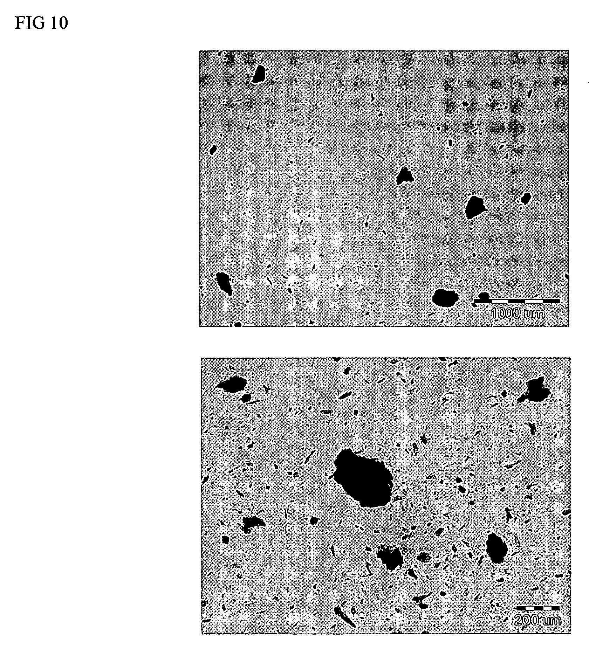

Figure 10: Hydrated form H as seen through a microscope

### Detailed Description of the Invention

According to a preferred embodiment of the present invention a process for preparing a compound represented by general formula 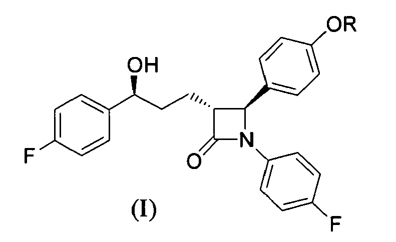 is provided, wherein R represents a hydrogen atom, a protective group selected from the group consisting of trisubstituted silyl, arylmethyl, mono or disubstituted arylmethyl with the substituents, elected from the group consisting of halides, methoxy, nitro, phenyl, naphthyl and any combinations thereof Said process comprises the steps of
a) metal-catalysed asymmetric transfer hydrogenation of p-fluoroacetophenones of general formula (II) 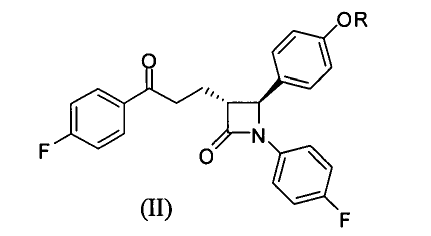 wherein R has the same meaning as above, i.e. R represents a hydrogen atom, a protective group selected from the group consisting of trisubstituted silyl, arylmethyl, mono or disubstituted arylmethyl with the substituents, elected from the group consisting of halides, methoxy, nitro, phenyl, naphthyl and any combinations thereof. The transfer hydrogenation is performed by using of a hydrogen donor in the presence of a metal catalyst based on Ruthenium complexes;
b) obtaining the compound represented by general formula (I); and
c) optionally purifying the compound represented by general formula (I).

According to an embodiment of the present invention R is selected from the group consisting of t-butyldimethylsilyl, t-butyldiphenylsilyl, triisopropylsilyl, trityl, benzyl, p-bromobenzyl, p-chlorobenzyl, p-nitrobenzyl, o-nitrobenzyl, p-phenylbenzyl, p-methoxybenzyl, characterised in that said process provides the compound represented by general formula (I) with a diastereometric ratio of more than 99:1.

According to another embodiment, R is selected from the group consisting of p-bromobenzyl, p-chlorobenzyl, p-nitrobenzyl, p-methoxybenzyl, trityl, tert-butyldimethylsilyl and benzyl.

According to still another embodiment of the present invention, the metal catalyst is based on Ruthenium complex of optically active N-sulfamoyl-1,2-diamine ligands of the general formula (VI): 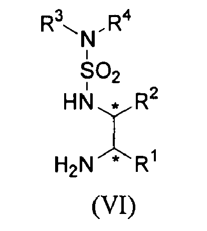 wherein:
- C* represents an asymmetric carbon atom;
- R¹ and R² independently represent a hydrogen atom, an optionally substituted aryl, or cycloalkyl, or R¹ and R² may be linked together to form a cyclohexane ring;
- R³ and R⁴ independently represent a hydrogen atom, a C₁₋₁₅ alkyl, linear or branched, optionally substituted with an aryl; or R³ and R⁴ may be linked together to form with the nitrogen atom an optionally substituted C₄₋₆ ring.

According to an embodiment, the optically active N-sulfamoyl-1,2-diamine ligands has enantiomer excess more than 99%.

According to an embodiment, R³ and/or R⁴ can be selected from the group consisting of methyl, iso-propyl and cyclohexyl.

According to another embodiment, R³ and R⁴ are linked together to form a ring selected from the group consisting of pyrrolidyl, piperidyl, morpholyl and azepanyl.

According to yet another embodiment, the metal catalyst is prepared from a ruthenium metal precursor and an optically active N-sulfamoyl-1,2-diamine ligand of the general formula (VI).

According to an embodiment of the present invention, the ruthenium catalyst precursor consists of η⁶-arene-ruthenium(II) halide dimers of the formula [RuX₂(η⁶-arene)]₂, wherein η⁶-arene represents an arene, selected from the group consisting of benzene, p-cymene, mesitylene, 1,3,5-triethylbenzene, hexamethylbenzene and anisole, and X is halide selected from the group consisting of chloride, bromide and iodide.

According to an embodiment, the hydrogen donor is based on HCO₂H. Examples for preferred hydrogen donors comprise HCO₂H-Et₃N, HCO₂H-iso-Pr₂NEt, HCO₂H-metal bicarbonates and HCO₂H-metal carbonates wherein the metal is selected from the group consisting of Na, K, Cs, Mg and Ca.

According to another embodiment, the metal-catalysed asymmetric transfer hydrogenation is conducted in solvent selected from the group consisting of dichloroethane, acetonitrile, N,N-dimethyl formamide, N,N-dimethylacetamide, 1-methyl-2-pyrrolidinone (NMP), 1,1,3,3-tetramethylurea, 1,3-dimethyl-2-imidazolidinone, N,N'-dimethylpropyleneurea and mixtures thereof.

According to a preferred embodiment, the compound of formula (I) is ezetimibe.

According to a preferred embodiment, ezetimibe form S specified by an X-ray powder diffraction pattern exhibiting peaks at the following diffraction angles: 7.3, 15.3, 16.7, 18.7, 21.8, 24.0°2Th is provided.

According to an embodiment, ezetimibe form S is specified by an X-ray powder diffraction pattern exhibiting additional peaks at the following diffraction angles: 6.2, 20, 25.3°2Th.

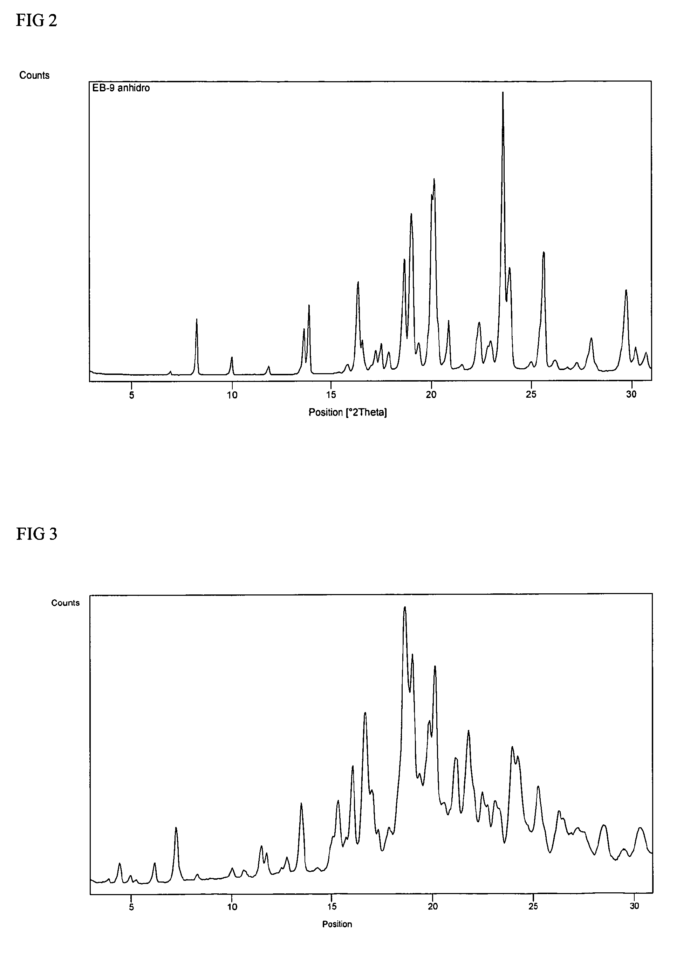

According to an embodiment, ezetimibe form S has an X-ray powder diffraction pattern as shown in Figure 3.

According to an embodiment, ezetimibe form S is specified by ¹H-NMR peaks at δ = 1.11 (s, t-Bu), 1.6-1.9 (m, 4 H, H-1', H-2'), 3.08 (m, 1 H, H-3), 4.20 (s, t-Bu-OH), 4.49 (m, 1 H, H-3'), 4.80 (d, J = 2.3 Hz, 1 H, H-4), 5.29 (br d, J = 2.7 Hz, 1 H, OH-3'), 6.73-6.78 (m, 2 H, Ar-H), 7.08-7.34 (m, 10 H, Ar-H), 9.54 (br s, 1 H, Ar-OH).

According to another embodiment, ezetimibe form S is characterized by a water content of about 0 to about 2%, determined by Karl Fischer analysis, a method well known to the skilled person. Preferably, the water content is about 0.5 to about 1.5% as determined by Karl Fischer analysis.

According to an embodiment, ezetimibe form S has a purity of more than 90%, preferably more than 99%, more preferably more than 99.6%.

According to an embodiment, ezetimibe form S contains from about 8 to about 15% of tert-butanol, preferably from about 10 to about 12% of tert-butanol.

According to another embodiment, the crystals of said ezetimibe form S have a particle size of less than about 100 microns, preferably less than 50 microns, more preferably less than about 30 microns.

According to still another embodiment, the micronized crystals of said ezetimibe form S have a particle size of less than about 30 microns, preferably less than 20 microns, more preferably less than about 10 microns.

According to an embodiment, ezetimibe form S contains not more than 20%, preferably not more than 10%, more preferably not more than 5% and most preferably not more than 1 % of other polymorphic forms.

According to another embodiment, the process for the preparation of ezetimibe form S comprises the steps of: a) dissolving ezetimibe of any polymorphic form in tert-butanol; b) cooling the resulting solution to room temperature; and c) drying.

According to a preferred embodiment a pharmaceutical composition comprising a therapeutically effective amount of ezetimibe is provided, which is prepared according to the present invention and optionally mixed with one or more active substances, and one or more pharmaceutically acceptable ingredients.

According to an embodiment, the pharmaceutical composition comprises a therapeutically effective amount of ezetimibe form S optionally mixed with one or more active substances, and one or more pharmaceutically acceptable ingredients.

According to another embodiment, the use of a therapeutically effective amount of ezetimibe is provided, wherein ezetimibe is prepared according to invention and is suitable for lowering cholesterol levels in a mammal in need of such treatment.

According to an embodiment, the use of a therapeutically effective amount of ezetimibe form S is provided for lowering cholesterol levels in mammal in need of such treatment.

According to a preferred embodiment, a compound of the formula 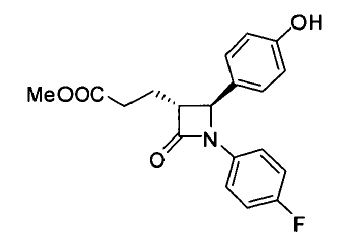 is provided.

According to another embodiment, a compound of the formula 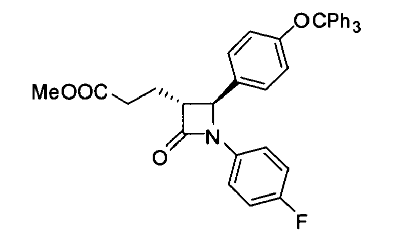 is provided.

According to still another embodiment, a compound of the formula 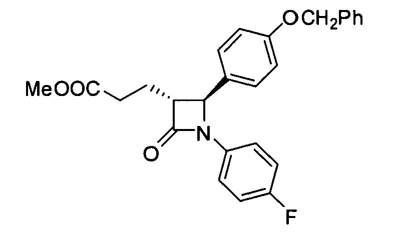 is provided.

In the present invention an efficient and effective preparation of ezetimibe (Ia) (formula (I) wherein R = H) in high yield is provided, involving the metal-catalyzed asymmetric transfer hydrogenation of p-fluoroacetophenones of general formula (II) to highly optically enriched alcohols preferably with a diastereomeric ratio (dr) of more than 99:1 of general formula (I), 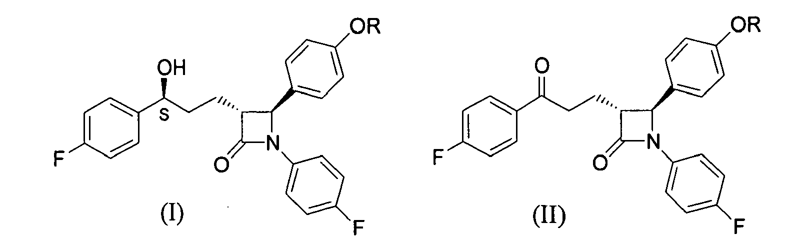 wherein, R represents a hydrogen atom (ezetimibe (Ia)), a protective group such as for example a trisubstituted silyl, triarylmethyl, arylmethyl, mono or disubstituted arylmethyl with the substituents, selected from the group consisting of halides (such as e.g. Cl, Br), methoxy, nitro, phenyl, naphthyl and any combinations thereof. Preferably R can be t-butyldimethylsilyl, t-butyldiphenylsilyl, triisopropylsilyl, trityl, benzyl, p-bromobenzyl, p-chlorobenzyl, p-nitrobenzyl, o-nitrobenzyl, p-phenylbenzyl, p-methoxybenzyl.

According to the present invention, the process relies on the use of a hydrogen donor in the presence of a metal catalyst based on ruthenium complexes of optically active (preferably >99% enantiomeric excess (ee)) N-sulfamoyl-1,2-diamine ligands (R³R⁴NSO₂-1,2-diamine) of the general formula (VI): 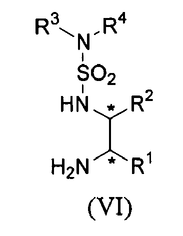 wherein:
- C* represents an asymmetric carbon atom;
- R¹ and R² independently represent a hydrogen atom, an optionally substituted aryl, or cycloalkyl, or R¹ and R² may be linked together to form a cyclohexane ring;
- R³ and R⁴ independently represent a hydrogen atom, a C₁₋₁₅ alkyl, linear or branched, optionally substituted with an aryl; preferably, R³ and/or R⁴ can be selected from the group consisting of methyl, iso-propyl, cyclohexyl; or R³ and R⁴ may be linked together to form with the nitrogen atom an optionally substituted C₄₋₆ ring such as e.g. pyrrolidyl, piperidyl, morpholyl, or azepanyl.

The optically active ruthenium complex is prepared from a ruthenium metal precursor and an optically active (preferably > 99% ee) N-sulfamoyl-1,2-diamine ligand of the general formula (VI) (wherein R¹, R², R³ and R⁴ are as defined above) and is used either in an isolated form or in situ. The ruthenium metal precursor consists of η⁶-arene-ruthenium(II) halide dimers of formula [RuX₂(η⁶-arene)]₂, wherein η⁶-arene represents an arene, selected from the group consisting of benzene, p-cymene, mesitylene, 1,3,5-triethylbenzene, hexamethylbenzene, anisole, and wherein X is a halide selected from the group consisting of chloride, bromide and iodide.

The metal-catalyzed asymmetric transfer hydrogenation according to the present invention can be carried out in the presence of a hydrogen donor known from literature as for example in Palmer et al. Tetrahedron: Asymmetry 1999, 10, 2045-2061. Preferably used are derivatives of HCO₂H such as e.g. HCO₂H-Et₃N, HCO₂H-iso-Pr₂NEt, HCO₂H-metal bicarbonates, HCO₂H-metal carbonates (the metal is selected from the group consisting of Na, K, Cs, Mg, Ca) and the like.

The Ruthenium catalyst used in the metal-catalysed asymmetric transfer hydrogenation according to the present invention can be obtained from the Ruthenium complex by activation in the presence of a base and/or the hydrogen donor.

Suitable solvents for the process of the present invention include but are not limited to solvents such as dichloroethane, acetonitrile, N,N-dimethyl formamide (DMF), N,N-dimethylacetamide (DMA), 1-methyl-2-pyrrolidinone (NMP), 1,1,3,3-tetramethylurea (TMU), 1,3-dimethyl-2-imidazolidinone (DMEU) N,N'-dimethylpropyleneurea (DMPU) and mixtures thereof.

The metal-catalysed asymmetric transfer hydrogenation may be conducted at reaction temperatures from about 15 °C to about 70 °C, preferably between about 30 °C to about 40 °C.

Surprisingly we found that the required amount of Ruthenium catalyst used in the process according to the present invention is low in comparison to the amount of other catalysts used in the syntheses of ezetimibe known from the prior art. The ruthenium catalyst may be used in an amount varying from about 0.05 to about 10 mol%, preferably between about 0.1 and about 1.0 mol%.

In yet another embodiment of the present invention, novel intermediates represented by the general formula (V) 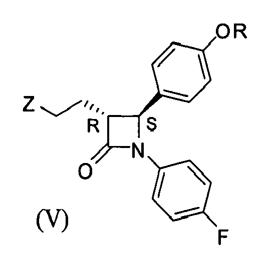 wherein:
- Z represents COCl, COOH, COOMe, CON(Me)OMe, or CON(Me)OEt;
- R represents a protective group as described previously for ketone (II). The R protecting group can be introduced by known methods as for example described by T.W. Greene and P.G.M. Wuts in "Protective Groups in Organic Synthesis", 1999, John Wiley & Sons. were prepared. The compounds are useful to easily access p-fluoroacetophenones of general formula (II).

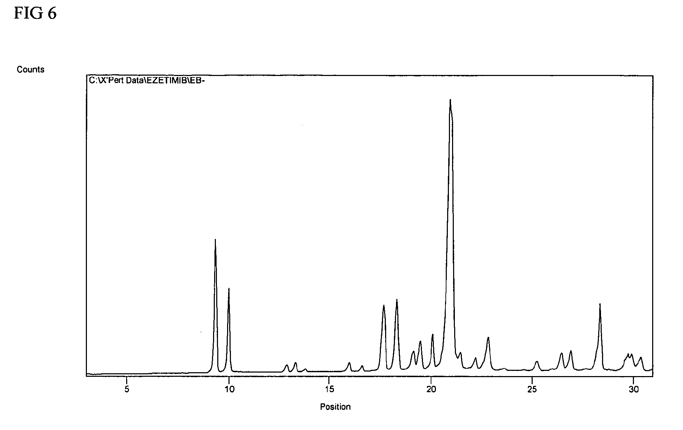

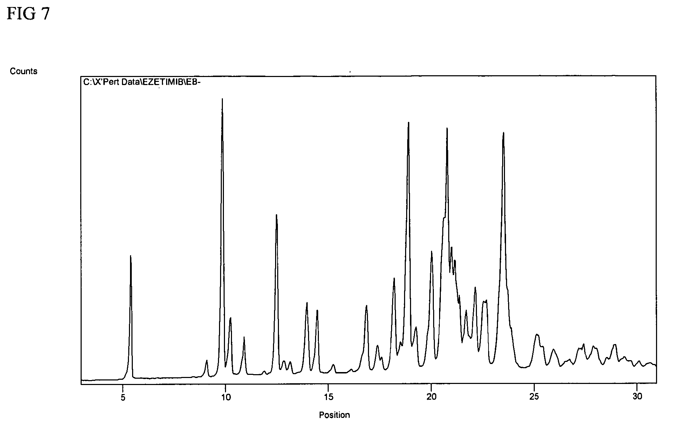

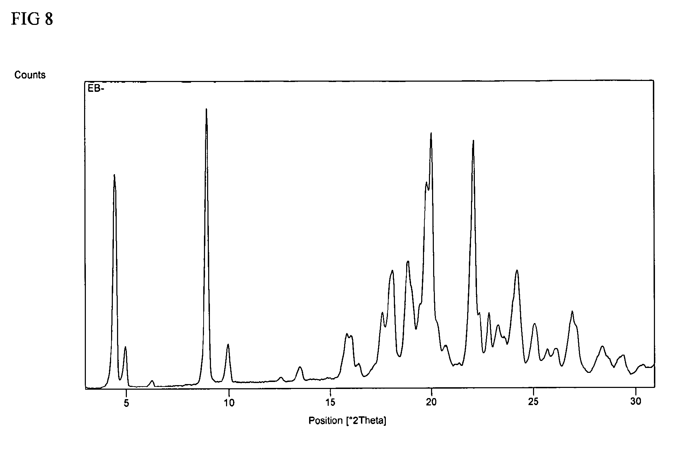

Examples for the compounds of the general formula (V) are:
Methyl 3-[(2S,3R)-1-(4-fluorophenyl)-2-(4-hydroxyphenyl)-4-oxoazetidinyl]propionate 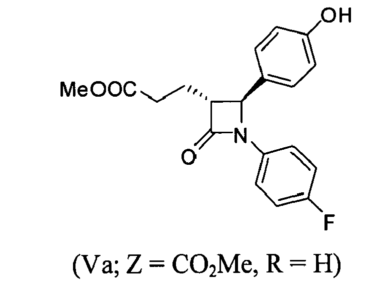 The powder X-ray diffraction pattern is illustrated in Figure 6.
Methyl 3-{(3R,4S)-1-(4-fluorophenyl)-2-oxo-4-[4-(trityloxy)phenyl]azetidin-3-yl}propionate 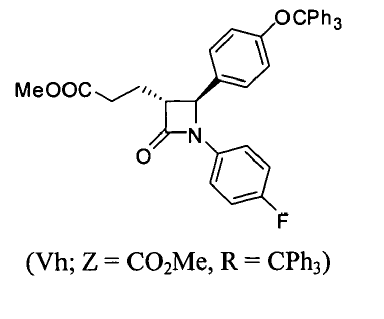 The powder X-ray diffraction pattern is illustrated in Figure 7.
Methyl 3-{(2S,3R)-2-[4-(benzyloxy)phenyl]-1-(4-fluorophenyl)-4-oxoazetidin-3-yl}-propionate 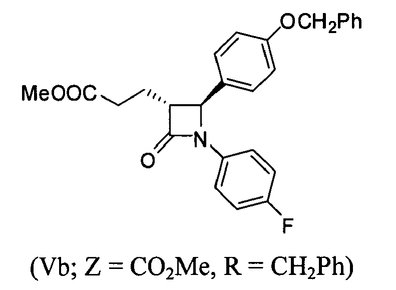 The powder X-ray diffraction pattern is illustrated in Figure 8.

The present compounds were characterized with regard to their melting points (T m) by means of Koffler melting point apparatus with accuracy of approximately ± 1°C and X-ray powder diffraction patterns (obtained by a Phillips PW3040/60 X'Pert PRO diffractometer using CuKα radiation of 1,541874 Å). Images of particles were taken on a Microscope Olympus BX 50 equipped with Olympus camera DP70.

The starting material used in the process of the present invention, which may be compound of general formula (Vb; Z = CO₂Me), may be hydrogenated in the presence of 10% Pd-C to yield the hydroxy deprotected compound of general formula (Va; Z = CO₂Me), which is further protected by a variety of reagents. The products of formula (Va; Z = CO₂Me), (Vb; Z = CO₂Me) and the O-trityl derivative of formula (Vh; Z = CO₂Me) are crystalline and are characterized by powder X-ray diffraction peaks:

| Va; Z = CO₂Me, R = H (°2Θ) | Vb; Z = CO₂Me, R = CH₂Ph (°2Θ) | Vh; Z = CO₂Me, R = CPh₃ (°2Θ) |
|---|---|---|
| 9.3 | 4.5 | 5.4 |
| 10.0 | 8.9 | 9.9 |
| 17.7 | 10.0 | 12.5 |
| 18.3 | 15.9 | 13.9 |
| 19.5 | 18,.1 | 16.9 |
| 20.1 | 18.9 | 18.2 |
| 21.0 | 20.0 | 18.9 |
| 22.9 | 22.0 | 20.0 |
| 28.4 | 24.2 | 20.7 |
| | 26.9 | 23.5 |

In the next step, the methyl ester moiety of compounds of general formula (Vb; Z = CO₂Me)-(Vk; Z = CO₂Me) is hydrolyzed to yield the free acids of general formula (Vb; Z = CO₂H)-(Vk; Z = CO₂H). The hydrolysis is carried out in the presence of a base, such as metal hydroxide, such as e.g. LiOH, NaOH, KOH, CsOH, Ca(OH)₂; quaternary ammonium hydroxide, e.g. benzyltrimethylammonium hydroxide; metal alkoxide, e.g. t-BuOK; metal carbonate, e.g. K₂CO₃, etc. Preferaby KOH is used. As solvents those with a low water content are preferably used, but not limited to, solvents such as THF, MeOH, EtOH, t-BuOH and mixtures thereof. The preferred solvents are THF and t-BuOH or any mixture thereof.

The obtained compounds of general formula (Vb; Z = CO₂H)-(Vk; Z = CO₂H) are activated by reacting them with oxalyl chloride to yield compounds of general formula (Vb; Z = COC1)-(Vk; Z = COC1).

Compounds of general formula (Vb; Z = COCI)-(Vk; Z = COC1) are then coupled with the in situ generated 4-fluorophenylzinc chloride to yield compounds of general formula (IIb)-(IIk).

Compounds of general formula (IIa)-(IIk) thus obtained as disclosed in Scheme 1.

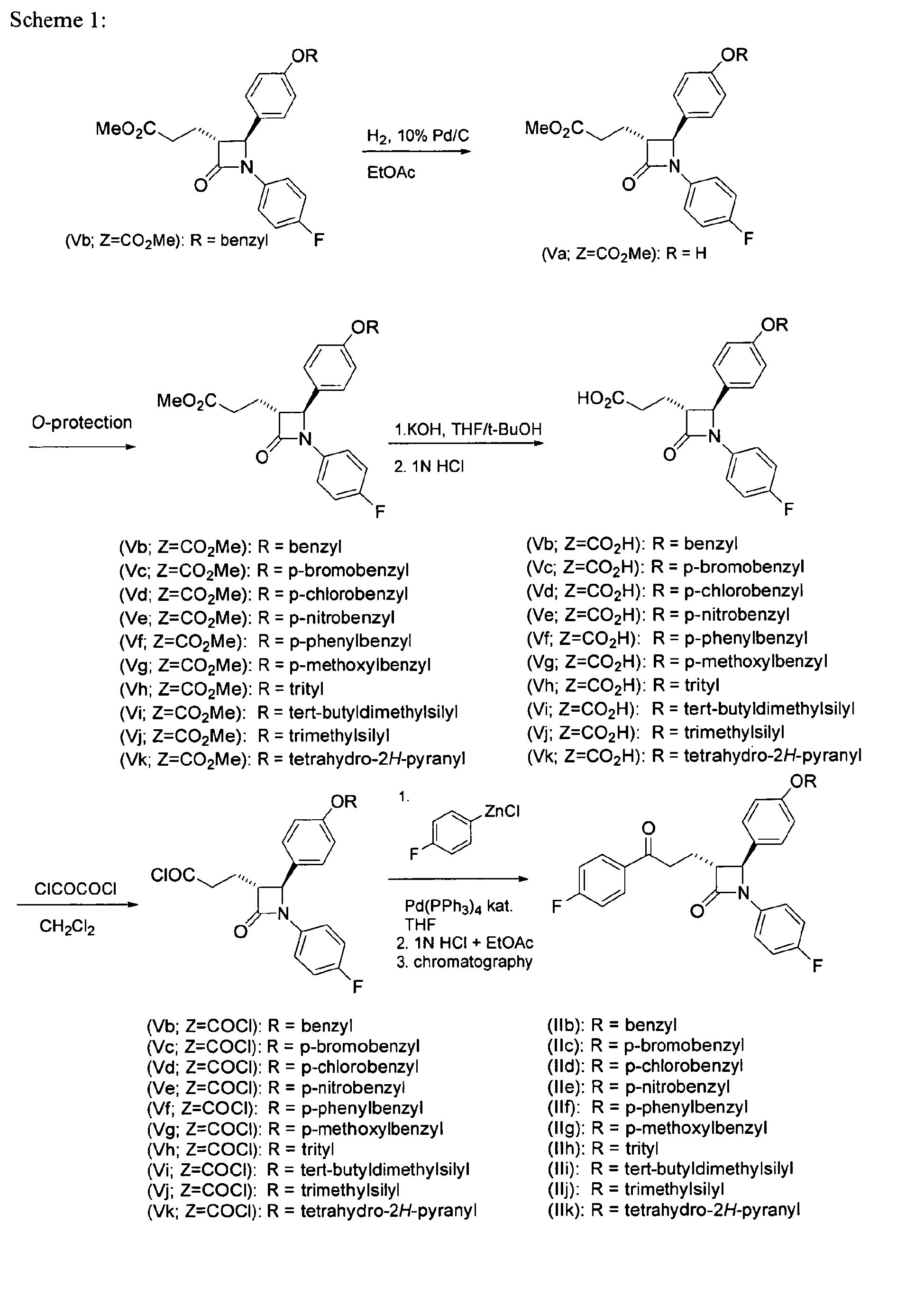

Said compounds are further reduced according to the present invention as disclosed in Scheme 2 yielding compounds of general formula (Ib)-(Ik).

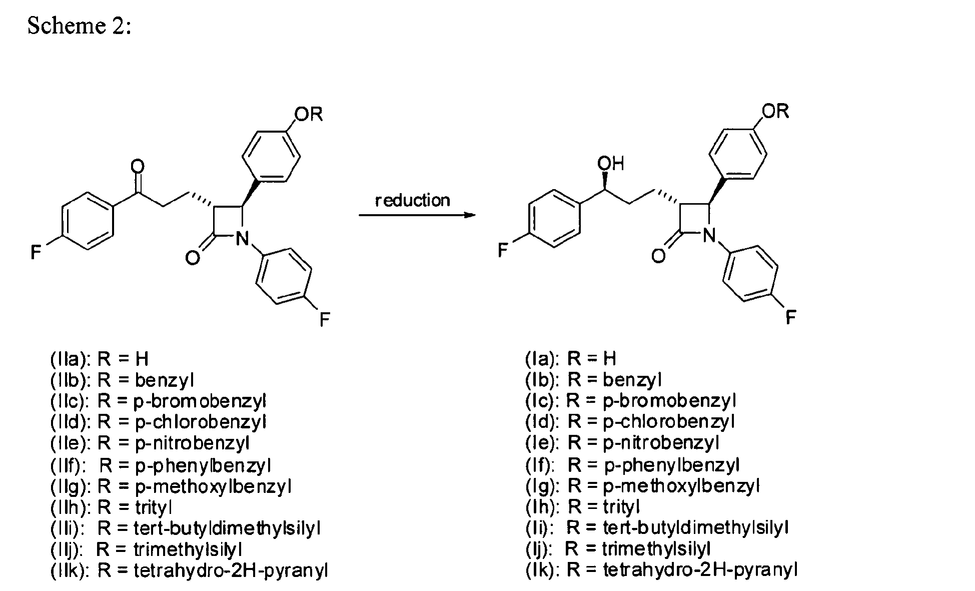

Deprotection of the group R of compounds of general formula (Ib)-(Ik) by any process known in the art, ezetimibe is obtained. In the preferred case, hydrogenation of compounds of general formula (Ib)-(Ih) in the presence of Pd/C or treatment of the compounds of general formula (Ih)-(Ik) with acidic reagents is used in the R group de-protection step.

Ezetimibe prepared according to the present invention have a purity of at least about 90%, more preferably at least about 95% and most preferably at least about 99% as measured by HPLC.

Ezetimibe prepared according to the process of the present invention can be isolated /crystallized or further purified by processes known from the prior art (as for example WO 2004/099132, WO 2005/066120, WO 2006/060808, WO 2005/062897, WO 2005/009955, WO 2006/050634, IPCOM000131677, G.Y.S.K.Swamy at all, Acta Cryst. (2005). E61, o3608-o3610). Solvents that could be used are n-butanol, n-propanol, chloroform, THF, acetone, bistrimethyl silyl acetamide, diethyl ketone, ethyl acetate, methanol and the like, particularly as for example isopropanol/water, methanol/water, ethanol/water etc.

Hydrated form of ezetimibe (marked as hydrated form H) characterized by powder X-ray diffraction peaks at 7.9; 15.8; 18.6; 19.3; 20.7; 21.7; 22.9; 23.4; 24.5; 25.2° 2Θ, is obtained when the crude ezetimibe is dissolved in a water containing solvent. It is further characterized by the water content of from about 4 to about 6%, preferably from about 4 to about 4.5% as determined by Karl Fischer analysis.

Anhydrous form A (marked as anhydro form A) characterized by powder X-ray diffraction peaks at 8.3; 13.9; 16.4; 18.7; 19.0; 20.1; 23.6; 23.9; 25.6; 29.7° 2Θ, is obtained when the crude ezetimibe is dissolved in an anhydrous solvent. Anhydro form A can be characterized by the water content of less than about 0.5%, preferably less than about 0.3% as determined by Karl Fischer analysis. Anhydro form A can be obtained by exposing the hydrated form H to relative humidity lower than about 20% for about 12 h, by drying of the hydrated form H in an air dryer at a relative humidity of less than about 50%, preferably less than about 40% and a temperature of about 30 to about 70 °C, preferably at about 40 to 50 °C, by vacuum drying at ambient temperature and pressure of less than about 100 mbar, or by crystallization of anhydro form A or hydrated form H from tert-butyl methyl ether/n-heptane.

The crystals of ezetimibe anhydro form A or hydrated form H can have a particle size of less than about 100 microns, preferably less than 50 microns, more preferably less than about 30 microns. Crystals of ezetimibe anhydro form A or hydrated form H may be further micronized by milling or any other process known from the prior art to obtain the micronized crystals of particle size of less than about 30 microns, preferably less than about 20 microns and more preferably less than about 10 microns.

Ezetimibe anhydro form A or hydrated form H is substantially free of other polymorphic forms, preferably it contains not more than 20%, more preferably not more than 10%, most preferably not more than 5% of other polymorphic forms.

While investigating the solubility of anhydro form A and hydrated form H in different solvents it was surprisingly found out that by using tert-butanol, known as anhydrous compound, as a solvent in the crystallization step a new form (named as form S) is obtained.

Another embodiment of the present invention is form S of ezetimibe characterized by powder X-ray diffraction peaks at the following diffraction angles

| No. | Pos. [°2Th.] | d-spacing [A] | Rel. Int. [%] |
|---|---|---|---|
| 1 | 6,2 | 14,21 | 11 |
| 2 | 7,3 | 12,15 | 23 |
| 3 | 15,3 | 5,78 | 32 |
| 4 | 16,7 | 5,31 | 63 |
| 5 | 18,7 | 4,75 | 100 |
| 6 | 20,1 | 4,41 | 80 |
| 7 | 21,8 | 4,08 | 57 |
| 8 | 24,0 | 3,71 | 51 |
| 9 | 25,3 | 3,52 | 37 |

Ezetimibe form S can be characterized by ¹H-NMR peaks at δ = 1.11 (s, t-Bu), 1.6-1.9 (m, 4 H, H-1', H-2'), 3.08 (m, 1 H, H-3), 4.20 (s, t-Bu-OH), 4.49 (m, 1 H, H-3'), 4.80 (d, J = 2.3 Hz, 1 H, H-4), 5.29 (br d, J = 2.7 Hz, 1 H, OH-3'), 6.73-6.78 (m, 2 H, Ar-H), 7.08-7.34 (m, 10 H, Ar-H), 9.54 (br s, 1 H, Ar-OH). NMR spectra were measured on a Varian Inova 300 MHz spectrometer in DMSO-d₆.

Ezetimibe form S of can be characterized by a water content of about 0 to about 2%, preferably of about 0.5 to about 1.5% determined by Karl Fischer (KF) analysis.

Yet another embodiment of the present invention is the process for the preparation of ezetimibe form S by dissolving anhydro form A and/or hydrated form B in tert-butanol. The resulting solution is cooled to room temperature and dried. In case that no precipitation takes place, the crystallization occurs after seeding with crystals of ezetimibe form S. The obtained ezetimibe form S has purity more than 90%, preferably more than 99%, more preferably more than 99.6%.

Ezetimibe form S contains from about 8 to about 15% of tert-butanol, preferably from about 10 to about 12% of tert-butanol. Ezetimibe form S may be further dried in order to be appropriate for incorporation into the pharmaceutical composition.

Crystals of ezetimibe form S are in the form of small particles and bigger agglomerates of irregular shape.

The crystals of ezetimibe form S can have a particle size of less than about 100 microns, preferably less than 50 microns, more preferably less than about 30 microns. Crystals of ezetimibe form S may be further micronized by milling or any other process known from the prior art to obtain the micronized crystals of particle size of less than about 30 microns, preferably less than about 20 microns and more preferably less than about 10 microns.

Ezetimibe form S is substantially free of other polymorphic forms. It contains not more than 20%, preferably not more than 10%, more preferably not more than 5%, most preferably not more than 1% of other polymorphic forms.

Surprisingly ezetimibe form S is stable upon drying, even at temperature of about 70°C only minimal loss of drying was observed in comparison to hydrated form H which is converted to anhydrous form A already at temperature of about 40°C. The stability to heating is very important factor in the preparation and storage of pharmaceutical compositions.

In another embodiment the present invention provides a pharmaceutical composition containing ezetimibe prepared according to the process of the present invention and being in any known polymorphic form as for example anhydro form A, hydrated form H or form S, optionally mixed with other active ingredients such as for example HMG-CoA reductase inhibitors and at least one pharmaceutical acceptable excipient.

The pharmaceutical composition according to the present invention can be in any conventional form, preferably an oral dosage form such as a capsule, tablet, pill, liquid, emulsion, granule, suppositories, powder, sachet, suspension, solution, injection preparation and the like. The formulations/compositions can be prepared using conventional pharmaceutically acceptable excipients. Such pharmaceutically available excipients and additives include fillers/diluents, binders, disintegrants, glidants, lubricants, wetting agents, preservatives, stabilizers, antioxidants, flavouring agents, coloring agents, emulsifier. Preferably, the oral dosage form is a tablet.

Suitable diluents include lactose, calcium carbonate, dibasic calcium phosphate anhydrous, dibasic calcium phosphate dehydrate (for example Emcompress^{®}), tribasic calcium phosphate, microcrystalline cellulose (such as for example Avicel^{®} PH 101, Avicel^{®} PH 102 etc), powdered cellulose, silicified microcrystalline cellulose (for example Prosolv^{®}), dextrates (for example Emdex^{®}), dextrose, fructose, glucose, lactitol, lactose anhydrous, lactose monohydrate, spray-dried lactose, magnesium oxide, magnesium carbonate, maltitol, maltodextrin, maltose, mannitol, starch, sucralose, sucrose, xilitol and others. Also, special excipients for direct compression such as cellactose or starlac can be used. Most often, lactose monohydrate and microcrystalline cellulose are used.

Binders are selected from the group consisting of gelatin, guar gum, cellulose derivatives (hydroxyethyl cellulose HEC, Hydroxyethylmethyl cellulose HEMC, hydroxypropyl cellulose HPC (for example Klucel^{®} EF or Klucel^{®} LF), hydroxypropylmethyl cellulose HPMC (Pharmacoat^{®} 603 or 606), methyl cellulose MC...), polymetacrylates, polyvinyl alcohol, povidone (of different grades, for example povidone K12, K15, K17, K25, K30 etc), starch and its derivatives (hydroxyethyl starch, pregelatinized starch) etc.

Suitable disintegrants include carboxymethyl cellulose sodium, carboxymethyl cellulose calcium, croscarmellose sodium, crospovidone, starch and modified starches (sodium starch glycolate - Primojel®), low substituted hydroxypropyl cellulose, magnesium aluminium silicate, calcium silicate and others.

Suitable surface active agents (solubilising agents) include, but are not limited to sodium laurylsulfate, glyceryl esters, polyoxyethylene glycol esters, polyoxyethylene glycol ethers, polyoxyethylene sorbitan fatty acid esters, sulphate containing surfactants, or polyoxyethylene/polyoxypropylene copolymers. The most preferred is sodium laurylsulfate.

Possible antioxidants include, but are not limited to, vitamin E acetate, α-tocopherol, ascorbyl palmitate, butylated hydroxyanisole (BHA), butylated hydroxytoluene (BHT), propyl gallate, dithiotreitol, or tocopherol polyethyleneglycol succinate (TPGS). Chelating agents can also be used as antioxidants, for example EDTA or cyclodextrins.

Suitable glidants are silicon dioxide, talc and aluminium silicate.

Lubricants are preferably selected from the group consisting of magnesium stearate, sodium stearyl fumarate, sucrose esthers of fatty acids, stearic acids and the like.

Sweeteners can be selected from aspartame, saccharin sodium, dipotassium glycirrhizinate, aspartame, thaumatin and the like.

The pharmaceutical composition according to the present invention may be prepared by well known technological processes such as direct compression or wet granulation, dry granulation or lyophilization. Preferably, wet granulation process in fluid bed system is used.

The ezetimibe prepared according to the present invention can be formulated in the pharmaceutical composition as described in WO 2007/003365.

The present invention is illustrated by the following examples without limiting it thereto.

### EXAMPLES

### Reference example (EP 0720599, example 6). Synthesis of ezetimibe from methyl 3-{(2S,3R)-2-[4-(benzyloxy)phenyl]-1-(4-fluorophenyl)-4-oxoazetidin-3-yl}propionate (Vb; Z = CO₂Me)

### Procedure 1:

To a solution of methyl 3-{(2S,3R)-2-[4-(benzyloxy)phenyl]-1-(4-fluorophenyl)-4-oxoazetidin-3-yl}propionate (Vb; Z = CO₂Me) (1.6 g, 3.7 mmol) in methanol (3.5 ml) and water (1.5 ml) was added lithium hydroxide monohydrate (155 mg, 3.7 mmol). The mixture was stirred at room temperature for 1.5 h, then additional amount of lithium hydroxide monohydrate (54 mg, 1.3 mmol) was added and stirring continued for 3 h. 1M hydrochloric acid (5 ml) and ethyl acetate (15 ml) were added, the organic layer was washed 3 times with water and dried over sodium sulfate. Concentration in vacuo afforded the acid (Vb; Z = CO₂H) (1.4 g, 89%) as an amber colored foam.

### Procedure 2:

To a solution of methyl 3-{(2S,3R)-2-[4-(benzyloxy)phenyl]-1-(4-fluorophenyl)-4-oxoazetidin-3-yl}propionate (Vb; Z = CO₂Me) (32 g, 74 mmol) in methanol (70 ml) and water (30 ml) was added lithium hydroxide monohydrate (3.1 g, 74 mmol). The mixture was stirred at room temperature for 1.5 h, then additional amount of lithium hydroxide monohydrate (1.08 g, 26 mmol) was added and stirring continued for 5.45 h. 1M hydrochloric acid (100 ml) and ethyl acetate (110 ml) were added, the organic layer was washed with water and dried over sodium sulfate. Concentration in vacuo afforded the acid (Vb; Z = CO₂H) (31.86 g, quant.) as an amber colored foam.

### Step 2

To a solution of 3-{(2S,3R)-2-[4-(benzyloxy)phenyl]-1-(4-fluorophenyl)-4-oxoazetidin-3-yl}propionic acid (Vb; Z = CO₂H) (30 g, 71.5 mmol) in dichloromethane (52 ml) was added 2M solution of oxalyl chloride in dichloromethane (53 ml, 106 mmol) and the mixture was stirred at room temperature for 16.5 h. Concentration in vacuo gave the acid chloride (Vb; Z = COCl) (31.45 g, quant.) as a viscous amber colored oil.

### Step 3

To a solution of dried zinc chloride (10.2 g, 73.4 mmol) in tetrahydrofuran (66 ml) was added dropwise 1M solution of 4-fluorophenylmagnesium bromide (73 ml) in tetrahydrofuran at 4 °C while stirring. Tetrakis(triphenylphosphine)palladium (4.12 g, 3.6 mmol) was added to the resulting suspension of 4-fluorophenylzinc chloride at 0 °C, followed by a solution of 3-{(2S,3R)-2-[4-(benzyloxy)phenyl]-1-(4-fluorophenyl)-4-oxoazetidin-3-yl}propionyl chloride (Vb; Z = COCl) (31.45 g, 71.5 mmol) in tetrahydrofuran (69 ml) and the cooling bath was removed. After 4.5 h of stirring 1M hydrochloric acid (20.5 ml) and ethyl acetate (200 ml) were added, the organic layer was washed with water (100 ml) and dried over sodium sulfate. Concentration afforded an oil which was purified by repeated silica gel chromatography with toluene/isopropanol (100/1). (3R,4S)-4-[4-(benzyloxy)phenyl]-1-(4-fluorophenyl-3-[3-(4-fluorophenyl)-3-oxopropyl]azetidin-2-one (IIb) (13.2 g, 37%) was obtained as a brown colored oil.

### Step 4

A solution of (3R,4S)-4-[4-(benzyloxy)phenyl]-1-(4-fluorophenyl)-3-[3-(4-fluorophenyl)-3-oxopropyl]azetidin-2-one (IIb) (6.54 g, 13 mmol) and (R)-1-methyl-3,3-diphenyltetrahydro-1H,3H-pyrrolo[1,2-c][1,3,2]oxazaborole (2.9 ml, 1M in toluene) in tetrahydrofuran (20.6 ml) was cooled to -20 °C, then borane-dimethylsulfide complex (2M in tetrahydrofuran; 5.85 ml, 11.7 mmol) was added dropwise over 1.5 h at -18 °C. The stirring was continued for additional 1 h at -19 °C, then methanol (3.5 ml) and 1M hydrochloric acid (27.5 ml) were carefully added. The mixture was extracted with ethyl acetate (41 ml), the organic layer was washed with water (2 x 48 ml) and dried over sodium sulfate. Concentration afforded crude (3R,4S)-4-[4-(benzyloxy)phenyl]-1-(4-fluorophenyl)-3-[(S)-3-(4-fluorophenyl)-3-hydroxypropyl]azetidin-2-one (Ib) (5.625 g, 86%) as a brown colored foam.

### Step 5

To a solution of (3R,4S)-4-[4-(benzyloxy)phenyl]-1-(4-fluorophenyl)-3-[(S)-3-(4-fluorophenyl)-3-hydroxypropyl]azetidin-2-one (Ib) (0.828 g, 1.66 mmol) in absolute ethanol (5.4 mL) was added 10 % palladium on carbon (62 mg, Heraeus). The reaction mixture was shaken in a pressure bottle under pressure of hydrogen gas (4 bar) for 40 h. Then additional amount of catalyst (62 mg) was added and the hydrogenolysis continued until reaction was estimated to be complete according to TLC analysis (toluene/ethyl acetate = 9/1). The catalyst was removed by filtration and washed with absolute ethanol (40 ml). The resulting solution was concentrated in vacuo to give crude ezetimibe (0.615 g, 90.7%) as a brownish solid. Immediate XRPD analysis showed the sample to be a mixture of amorphous and crystalline phases (anhydro < hydrated). Reanalysis after 9 days revealed slightly lesser amount of amorphous phase and prevalance of the anhydro over hydrated form in the crystalline phase. 0.438 g of this material was purified by recrystallization from ethanol/water (5/1, 3.7 ml). After stirring at room temperature for cca. 80 min and cooling for 15 min in an ice bath the crystals were filtered and washed with cold ethanol/water (1/1) mixture (6 ml) to yield ezetimibe (0.276 g) in hydrated form H according to XRPD analysis, which had mp 159-161.5 °C.

### Hydrolysis step (Step 1) optimization

### Example 1. 3-{(2S,3R)-2-[4-(benzyloxy)phenyl]-1-(4-fluorophenyl)-4-oxoazetidin-3-yl}propionic acid (Vb; Z = CO₂H)

### Procedure 1: Hydrolysis with KOH in THF/t-BuOH = 1/3, small scale

To a solution of methyl 3-{(2S,3R)-2-[4-(benzyloxy)phenyl]-1-(4-fluorophenyl)-4-oxoazetidin-3-yl}propionate (Vb; Z = CO₂Me) (1.6 g, 3.7 mmol) in tetrahydrofuran (1 ml) and tert-butanol (3 ml) was added powdered potassium hydroxide (244 mg, 3.7 mmol). The mixture was stirred at room temperature for 1 h, then additional amount of powdered potassium hydroxide (90 mg, 1.3 mmol) was added and stirring continued for 1 h. 1M hydrochloric acid (5 ml) and ethyl acetate (18 ml) were added, the organic layer was washed 3 times with water and dried over sodium sulfate. Concentration in vacuo afforded the acid (Vb; Z = CO₂H) (1.5 g, 95%) as a viscous oil.

### Procedure 2: Hydrolysis with KOH in THF/t-BuOH = 1/3, larger scale

To a solution of methyl 3-{(2S,3R)-2-[4-(benzyloxy)phenyl]-1-(4-fluorophenyl)-4-oxoazetidin-3-yl}propionate (Vb; Z = CO₂Me) (32 g, 74 mmol) in tetrahydrofuran (20 ml) and tert-butanol (60 ml) was added powdered potassium hydroxide (4.88 g, 74 mmol). The mixture was stirred at room temperature for 1.5 h. Then 1M hydrochloric acid (100 ml) and ethyl acetate (200 ml) were added. The organic layer was washed 3 times with water and dried over sodium sulfate. Concentration in vacuo afforded the acid (Vb; Z = CO₂H) (30.9 g, 99%) as an amber foam.

### Procedure 3: Hydrolysis with t-BuOK + H₂O in THF

To a solution of methyl 3-{(2S,3R)-2-[4-(benzyloxy)phenyl]-1-(4-fluorophenyl)-4-oxoazetidin-3-yl}propionate (Vb; Z = CO₂Me) (100 mg, 0.231 mmol) in tetrahydrofuran (2 ml) and water (10 mg, 0.462 mmol) was added potassium tert-butoxide (0.54 g, 1.85 mmol). The resulting suspension was stirred at room temperature for 72 h, then 1M hydrochloric acid (2 ml) and diethyl ether (20 ml) were added. The organic layer was washed 3 times with water and dried over sodium sulfate. Concentration in vacuo afforded the acid (Vb; Z = CO₂H) (86 mg, 89%) as a viscous oil.

### Procedure 4: Hydrolysis with t-BuOK in H₂O/ THF mixture

To a solution of methyl 3-{(2S,3R)-2-[4-(benzyloxy)phenyl]-1-(4-fluorophenyl)-4-oxoazetidin-3-yl}propionate (Vb; Z = CO₂Me) (50 mg, 0.146 mmol) in tetrahydrofuran (4 ml) and water (53 mg, 2.92 mmol) was added potassium tert-butoxide (0.54 g, 1.85 mmol). The resulting suspension was stirred at room temperature for 1 h, then heated to 50 °C for additional 1.5 h. After cooling to room temperature, 0.5M hydrochloric acid (2 ml) and diethyl ether (10 ml) were added to the reaction mixture. The organic layer was dried over sodium sulfate and concentrated in vacuo to afford the acid (Vb; Z = CO₂H) (45 mg, 92%) as a viscous oil.

### Procedure 5: Hydrolysis with KOH in THF

To a solution of methyl 3-{(2S,3R)-2-[4-(benzyloxy)phenyl]-1-(4-fluorophenyl)-4-oxoazetidin-3-yl}propionate (Vb; Z = CO₂Me) (50 mg, 0.146 mmol) in tert-butanol (1.5 ml) was added powdered potassium hydroxide (13 mg, 0.232 mmol). The mixture was stirred at room temperature for 2 h, then 0.5M hydrochloric acid (2 ml) and tert-butyl methyl ether (15 ml) were added. The organic layer was washed 3 times with water, dried over sodium sulfate and concentrated in vacuo to obtain the acid (Vb; Z = CO₂H (45 mg, 92%) as a viscous oil.

### Hydrolysis step (Step 1) optimization. Hydrolysis with KOH in THF/t-BuOH, other examples

### Example 2. 3-{(2S,3R)-2-[4-(4-bromobenzyloxy)phenyl]-1-(4-fluorophenyl)-4-oxoazetidin-3-yl}propionic acid (Vc; Z = CO₂H)

To a solution of methyl 3-{(2S,3R)-2-[4-(4-bromobenzyloxy)phenyl]-1-(4-fluorophenyl)-4-oxoazetidin-3-yl}propionate (Vc; Z = CO₂Me) (11.5 g, 22.4 mmol) in tetrahydrofuran (27 ml) and tert-butanol (36 ml) was added powdered potassium hydroxide (1.75 g, 22.4 mmol). The mixture was stirred at room temperature for 0.5 h, then 1M hydrochloric acid (30 ml) and ethyl acetate (100 ml) were added. The organic layer was washed 3 times with water and dried over sodium sulfate. Concentration in vacuo afforded the acid (Vc; Z = CO₂H) (9.2 g, 82%) as an almost colorless foam.

### Example 3. 3-{(2S,3R)-2-[4-(4-chlorobenzyloxy)phenyl]-1-(4-fluorophenyl)-4-oxoazetidinyl}propionic acid (Vd; Z = CO₂H)

To a solution of methyl 3-{(2S,3R)-2-[4-(4-chlorobenzyloxy)phenyl]-1-(4-fluorophenyl)-4-oxoazetidin-3-yl}propionate (Vd; Z = CO₂Me) (12.78 g, 27.3 mmol) in tetrahydrofuran (30 ml) and tert-butanol (90 ml) was added powdered potassium hydroxide (1.95 g, 34.8 mmol). The mixture was stirred at room temperature for 6 h, then 1M hydrochloric acid (40 ml) and ethyl acetate (100 ml) were added. The organic layer was washed with water and dried over sodium sulfate. Concentration in vacuo afforded the acid (Vd; Z = CO₂H) (13.1 g, 96%) as an almost colorless oil.

### Example 4. 3-{(2S,3R)-1-(4-fluorophenyl)-2-[4-(4-methoxybenzyloxy)phenyl]-4-oxoazetidin-3-yl}propionic acid (Vg; Z = CO₂H)

To a solution of methyl 3-{(2S,3R)-1-(4-fluorophenyl)-2-[4-(4-methoxybenzyloxy)phenyl]-4-oxoazetidin-3-yl}propionate (Vg; Z = CO₂Me) (2.7 g, 4.31 mmol) in tetrahydrofuran (2 ml) and tert-butanol (6 ml) was added powdered potassium hydroxide (0.48 g, 8.63 mmol). The mixture was stirred at room temperature for 0.5 h, then 1M hydrochloric acid (10 ml) and ethyl acetate (20 ml) were added. The organic layer was washed with water and dried over sodium sulfate. Concentration in vacuo afforded the acid (Vg; Z = CO₂H) (1.78 g, 94%) as a yellow colored viscous oil.

### Example 5. 3-{(3R,4S)-1-(4-fluorophenyl)-2-oxo-4-[4-(trityloxy)phenyl]azetidin-3-yl}propionic acid (Vh; Z = CO₂H)

To a solution of methyl 3-{(3R,4S)-1-(4-fluorophenyl)-2-oxo-4-[4-(trityloxy)phenyl]azetidin-3-yl}propionate (Vh; Z = CO₂Me) (6.0 g, 8.9 mmol) in tetrahydrofuran (10 ml) and tert-butanol (20 ml) was added powdered potassium hydroxide (0.6 g, 8.9 mmol). The mixture was stirred at room temperature for 19 h, then additional amount of powdered potassium hydroxide (0.15 g, 2.2 mmol) was added and stirring continued for 2 h. 1M hydrochloric acid (5 ml) and ethyl acetate (18 ml) were added, the organic layer was washed with water and dried over sodium sulfate. Concentration in vacuo afforded the acid (Vh; Z = CO₂H (5.2 g, 88%) as an almost colorless foam.

### Chlorination with oxalyl chloride (Step 2)

### Example 6. 3-{(2S,3R)-2-[4-(4-bromobenzyloxy)phenyl]-1-(4-fluorophenyl)-4-oxoazetidin-3-yl}propionyl chloride (Vc; Z = COCl)

To a solution of 3-{(2S,3R)-2-[4-(4-bromobenzyloxy)phenyl]-1-(4-fluorophenyl)-4-oxoazetidin-3-yl}propionic acid (Vc; Z = CO₂H) (9.2 g, 18.4 mmol) in dichloromethane (16 ml) was added 2M solution of oxalyl chloride (14 ml, 28.0 mmol) in dichloromethane and the mixture was stirrred at room temperature for 18 h. Concentration in vacuo gave the acid chloride (Vc; Z = COCl) as a viscous amber colored oil.

### Example 7. 3-{(2S,3R)-2-[4-(4-chlorobenzyloxy)phenyl]-1-(4-fluorophenyl)-4-oxoazetidin-3-yl}propionyl chloride (Vd; Z = COCl)

To a solution of 3-{(2S,3R)-2-[4-(4-chlorobenzyloxy)phenyl]-1-(4-fluorophenyl)-4-oxoazetidin-3-yl}propionic acid (Vd; Z = CO₂H) (13.0 g, 27.8 mmol) in dichloromethane (37 ml) was added 2M solution of oxalyl chloride (22.7 ml, 45.4 mmol) in dichloromethane and the mixture was stirrred at room temperature for 18 h. Concentration in vacuo gave the acid chloride (Vd; Z = COCl) as a viscous brown colored oil.

### Example 8. 3-{(2S,3R)-1-(4-fluorophenyl)-2-[4-(4-methoxybenzyloxy)phenyl]-4-oxoazetidin-3-yl}propionyl chloride (Vg; Z = COCl)

To a solution of 3-{(2S,3R)-1-(4-fluorophenyl)-2-[4-(4-methoxybenzyloxy)phenyl]-4-oxoazetidin-3-yl}propionic acid (Vg; Z = CO₂H) (2.6 g, 5.74 mmol) in dichloromethane (7 ml) was added 2M solution of oxalyl chloride (4.4 ml, 8.8 mmol) in dichloromethane and the mixture was stirrred at room temperature for 18 h. Concentration in vacuo gave the acid chloride (Vg; Z = COCl) as a viscous amber colored oil.

### Example 9. 3-{(3R,4S)-1-(4-fluorophenyl)-2-oxo-4-[4-(trityloxy)phenyl]azetidin-3-yl}propionyl chloride (Vh; Z = COCl)

To a solution of 3-{(3R,4S)-1-(4-fluorophenyl)-2-oxo-4-[4-(trityloxy)phenyl] azetidin-3-yl}propionic acid (Vh; Z = CO₂H) (5.1 g, 7.7 mmol) in dichloromethane (15 ml) was added 2M solution of oxalyl chloride (5.7 ml, 11.4 mmol) in dichloromethane and the mixture was stirrred at room temperature for 18 h. Concentration in vacuo gave the acid chloride (Vh; Z = COCl) as a viscous yellow colored oil.

### Coupling of acid chlorides with 4-fluorophenylzinc chloride (Step 3)

### Example 10. (3R,4S)-4-[4-(4-bromobenzyloxy)phenyl]-1-(4-fluorophenyl)-3-[3-(4-fluorophenyl)-3-oxopropyl]azetidin-2-one (IIc)

To a solution of dried zinc chloride (2.68 g, 19.4 mmol) in tetrahydrofuran (20 ml) was added dropwise 1M solution of 4-fluorophenylmagnesium bromide in tetrahydrofuran (19.4 ml) at 4 °C while stirring. Tetrakis(triphenylphosphine)palladium (1.1 g, 0.96 mmol) was added to the resulting suspension of 4-fluorophenylzinc chloride at 10 °C, followed by a solution of 3-{(2S,3R)-2-[4-(4-bromobenzyloxy)phenyl]-1-(4-fluorophenyl)-4-oxoazetidin-3-yl}propionyl chloride (Vc; Z = COCl) (9.2 g, 17.4 mmol) in tetrahydrofuran (17 ml) and the cooling bath was removed. After 7 h of stirring, 1M hydrochloric acid (17 ml) and ethyl acetate (250 ml) were added, the organic layer was washed with water and dried over sodium sulfate. Concentration afforded an oil (9.5 g) which was purified by silica gel chromatography with toluene/isopropanol (200/1). Ketone IIc was obtained as an amber colored oil.

### Example 11. (3R,4S)-4-[4-(4-chlorobenzyloxy)phenyl]-1-(4-fluorophenyl-3-[3-(4-fluorophenyl)-3-oxopropyl]azetidin-2-one (IId)

To a solution of dried zinc chloride (3.88 g, 27 mmol) in tetrahydrofuran (25 ml) was added dropwise 1M solution of 4-fluorophenylmagnesium bromide in tetrahydrofuran (72 ml) at 0 °C while stirring. Tetrakis(triphenylphosphine)palladium (1.57 g, 1.36 mmol) was added to the resulting suspension of 4-fluorophenylzinc chloride (27 mmol) at 4 °C, followed by a solution of 3-{(2S,3R)-2-[4-(4-chlorobenzyloxy)phenyl]-1-(4-fluorophenyl)-4-oxoazetidin-3-yl}propionyl chloride (Vd; Z = COCl) (12.0 g, 25 mmol) in tetrahydrofuran (20 ml) and the cooling bath was removed. After 3 h of stirring 1M hydrochloric acid (25 ml) and ethyl acetate (100 ml) were added, the organic layer was washed with water and dried over sodium sulfate. Concentration afforded a brown colored oil (12.7 g) which was purified by silica gel chromatography with toluene/isopropanol (200/1). Ketone IId was obtained as a brown colored oil.

### Example 12. (3R,4S)-1-(4-fluorophenyl)-3-[3-(4-fluorophenyl)-3-oxopropyl]-4-[4-(4-methoxybenzyloxy)phenyl]azetidin-2-one (IIg)

To a solution of dried zinc chloride (0.47 g, 3.5 mmol) in tetrahydrofuran (3.5 ml) was added dropwise 1M solution of 4-fluorophenylmagnesium bromide in tetrahydrofuran (3.5 ml) at 4 °C while stirring. Tetrakis(triphenylphosphine)palladium (0.19 g, 0.16 mmol) was added to the resulting suspension of 4-fluorophenylzinc chloride at 0 °C, followed by a solution of 3-{(2S,3R)-1-(4-fluorophenyl)-2-[4-(4-methoxybenzyloxy)phenyl]-4-oxoazetidin-3-yl}propionyl chloride (Vg; Z = COCl) (1.5 g, 2.89 mmol) in tetrahydrofuran (3.2 ml) and the cooling bath was removed. After 4 h of stirring 1M hydrochloric acid (2.3 ml) and ethyl acetate (50 ml) were added, the organic layer was washed with water, dried over sodium sulfate and concentrated to obtain the crude product IIg (1.1 g) as a brown colored oil.

### Example 13. (3R,4S)-1-(4-fluorophenyl)-3-[3-(4-fluorophenyl)-3-oxopropyl]-4-[4-(trityloxy)phenyl]azetidin-2-one (IIh)

To a solution of dried zinc chloride (1.02 g, 7.3 mmol) in tetrahydrofuran (7 ml) was added dropwise 1M solution of 4-fluorophenylmagnesium bromide in tetrahydrofuran (7.3 ml) at 0 °C while stirring. Tetrakis(triphenylphosphine)palladium (0.41 g, 0.35 mmol) was added to the resulting suspension of 4-fluorophenylzinc chloride (7.3 mmol) at 4 °C, followed by a solution of 3-{(3R,4S)-1-(4-fluorophenyl)-2-oxo-4-[4-(trityloxy)phenyl]azetidin-3-yl}propionyl chloride (Vh; Z = COCl) (4.0 g, 7.0 mmol) in tetrahydrofuran (3.2 ml) and the cooling bath was removed. After 3 h of stirring 0.1M acetic acid (20 ml) and ethyl acetate (50 ml) were added, the organic layer was washed with water, dried over sodium sulfate and concentrated to obtain the product IIh (3.43 g) as a brown oil.

### Transfer hydrogenation of ketones II (Step 4)

### Example 14: Transfer hydrogenation of ketone (IIb)

The Ru-complex was prepared from [RuCl₂(mesitylene)]₂ (11.5 mg, 40 µmol Ru at.) and (1*S*,2*S*)-N-piperidylsulfamoyl-1,2-diphenylethylenediamine (17 mg, 48 µmol) by heating in acetonitrile (2 ml) at 80 °C for 30 min. The Ru-complex solution and HCO₂H-Et₃N (5:2, 2 ml) were then added over 24 h in 5 portions to (IIb) (2.50 g, 5.0 mmol) in acetonitrile (5 ml) stirred at 40 °C. The mixture was partitioned between ethyl acetate (20 ml) and water (20 ml), the organic layer washed with brine (20 ml), dried over Na₂SO₄, and filtered through a bed of silica gel. The residue of concentration was recrystallized from iso-propyl ether, then from ethanol to afford 2.27 g (90.5%) of the alcohol (Ib) with dr = 94:6 (determined by ¹⁹F NMR (CDCl₃) using 1.5 mol equiv. Eu(hfc)₃).

### Example 15: Transfer hydrogenation of ketone (IIa)

The Ru-complex was prepared from [RuCl₂(mesitylene)]₂ (2.1 mg, 7.2 µmol Ru at.) and (1*S*,2*S*)-N-piperidylsulfamoyl-1,2-diphenylethylenediamine (3.2 mg, 8.9 µmol) by heating in (CH₂Cl)₂ (0.5 ml) at 80 °C for 30 min. The Ru-complex solution and HCO₂H-Et₃N (5:2, 210 µl) were then added in portions over 24 h to (IIa) (150 mg, 0.37 mmol) in (CH₂Cl)₂ (0.5 ml) stirred at 40 °C. The mixture was partitioned between ethyl acetate (5 ml) and water (5 ml), the organic layer washed with brine (5 ml), dried over Na₂SO₄, and filtered through a bed of silica gel. The residue of concentration (134 mg) was recrystallized from ethanol-water (4:1) to afford the product (Ia) with dr > 99:1 (determined by ¹⁹F NMR (CDCl₃) using 1.5 mol equiv. Eu(hfC)₃).

### O-Deprotection of alcohols I (Step 5)

### Example 16: O-Deprotection of alcohol (Ib)

A mixture of alcohol (Ib) (2.19 g, 4.38 mmol, dr = 94:6) and 10% Pd-C (160 mg) in ethanol/ethyl acetate (2:1, 35 ml) was hydrogenated at 30 psi of H₂ for 10 h and then filtered through Celite. The residue of concentration was recrystallized from ethanol-water (4:1) to afford 1.27 g (71%) of product (Ia) melting from 160 to 163 °C with dr > 99:1 (determined by ¹⁹F NMR (CDCl₃) using 1.5 mol equiv. Eu(hfc)₃).

### Preparation of starting materials

### Example 17. Methyl 3-[(2S,3R)-1-(4-fluorophenyl)-2-(4-hydroxyphenyl)-4-oxoazetidin-3-yl]propionate (Va; Z = CO₂Me)

To a solution of methyl 3-{(2S,3R)-2-[4-(benzyloxy)phenyl]-1-(4-fluorophenyl)-4-oxoazetidin-3-yl}propionate (Vb; Z = CO₂Me) (50 g, 115 mmol) in ethyl acetate (90 mL) was added 10% palladium on carbon (4 g, with 49.6% of water, Engelhard). The reaction mixture was shaken in a pressure bottle under the pressure of hydrogen gas (3.5 bar) for 20 h. Catalyst was removed by filtration through a filter aid and washed with ethyl acetate (10 ml). The resulting solution was dried over sodium sulfate and concentrated. The solid residue was purified by recrystallization in methanol/water (5/1) to yield the ester (Va; Z = CO₂Me) (38.4 g, 97%) as a white solid with m. p. 136-137 °C.

### Example 18. Methyl 3-{(2S,3R)-2-[4-(4-bromobenzyloxy)phenyl]-1-(4-fluorophenyl)-4-oxoazetidin-3-yl}propionate (Vc; Z = CO₂Me)

A mixture of methyl 3-[(2S,3R)-1-(4-fluorophenyl)-2-(4-hydroxyphenyl)-4-oxoazetidin-3-yl]propionate (Va; Z = CO₂Me) (10.0 g, 29 mmol), 4-bromobenzyl bromide (11.0 g, 41 mmol), anhydrous potassium carbonate (40.0 g, 0.29 mol) and tetrabutylammonium iodide (1.00 g, 3 mmol) in acetone (30 ml) was stirred under reflux for 3.5 h. The mixture was cooled to room temperature, filtered and concentrated in vacuo. The residue was purified by silica gel chromatography with toluene/ethyl acetate (5/1) to give pure ester (Vc; Z = CO₂Me) (11.5 g, 85%) as a white colored solid.

### Example 19. Methyl 3-{(2S,3R)-2-[4-(4-chlorobenzyloxy)phenyl]-1-(4-fluorophenyl)-4-oxoazetidin-3-yl}propionate (Vd; Z = CO₂Me)

A mixture of methyl 3-[(2S,3R)-1-(4-fluorophenyl)-2-(4-hydroxyphenyl)-4-oxoazetidin-3-yl]propionate (Va; Z = CO₂Me) (1 g, 2.9 mmol), 4-chlorobenzyl chloride (0.7 g, 3.2 mmol), anhydrous potassium carbonate (2 g, 15 mmol) and tetrabutylammonium iodide (0.01 g, 0.03 mmol) in acetone (4 ml) was stirred under reflux for 5 h. The mixture was cooled to room temperature, filtered and concentrated in vacuo. The residue was purified by silica gel chromatography with toluene/ethyl acetate (3/1) to give pure ester (Vd; Z = CO₂Me) (0.8 g, 60%) as an amber colored oil.

### Example 20. Methyl 3-{(2S,3R)-1-(4-fluorophenyl)-2-[4-(4-nitrobenzyloxy)phenyl]-4-oxoazetidin-3-yl}propionate (Ve; Z = CO₂Me)

A mixture of methyl 3-[(2S,3R)-1-(4-fluorophenyl)-2-(4-hydroxyphenyl)-4-oxoazetidin-3-yl]propionate (Va; Z = CO₂Me) (100 mg, 0.29 mmol), 4-nitrobenzyl chloride (63 mg, 0.364 mmol), anhydrous potassium carbonate (102 mg, 0.56 mmol) and tetrabutylammonium iodide (18 mg, 0.05 mmol) in acetone (4 ml) was stirred under reflux for 7 h. The mixture was cooled to room temperature, filtered and concentrated in vacuo. The residue was purified by silica gel chromatography with toluene/ethyl acetate (9/1) to give pure ester (Ve; Z = CO₂Me) (69 mg, 50%) as an amber colored oil.

### Example 21. Methyl 3-{(2S,3R)-2-[4-(biphenyl-4-ylmethoxy)phenyl]-1-(4-fluorophenyl)-4-oxoazetidin-3-yl}propionate (Vf; Z = CO₂Me)

A mixture of methyl 3-[(2S,3R)-1-(4-fluorophenyl)-2-(4-hydroxyphenyl)-4-oxoazetidin-3-yl]propionate (Va; Z = CO₂Me) (1.0 g, 2.9 mmol), 4-phenylbenzyl chloride (0.61 g, 3.0 mmol), anhydrous potassium carbonate (1.0 g, 7.2 mmol) and tetrabutylammonium iodide (0.01 g, 0.03 mmol) in acetone (10 ml) was stirred under reflux for 5 h. The mixture was cooled to room temperature, filtered and concentrated in vacuo. The residue was purified by silica gel chromatography with toluene/ethyl acetate (9/1) to give pure ester (Vf; Z = CO₂Me) (0.80 g, 60%) as a brown colored oil.

### Example 22. Methyl 3-{(2S,3R)-1-(4-fluorophenyl)-2-[4-(4-methoxybenzyloxy)phenyl]-4-oxoazetidin-3-yl}propionate (Vg; Z = CO₂Me)

A mixture of methyl 3-[(2S,3R)-1-(4-fluorophenyl)-2-(4-hydroxyphenyl)-4-oxoazetidin-3-yl]propionate (Va; Z = CO₂Me) (100 mg, 0.29 mmol), 4-methoxybenzyl bromide (75 mg, 0.364 mmol), anhydrous potassium carbonate (200 mg, 1.45 mmol) and tetrabutylammonium iodide (10 mg, 0.03 mmol) in acetone (1 ml) was stirred under reflux for 4 h. The mixture was cooled to room temperature, filtered and concentrated in vacuo. The residue was purified by silica gel chromatography with toluene/ethyl acetate (9/1) to give pure ester (Vg; Z = CO₂Me) (105 mg, 78%).

### Example 23. Methyl 3-{(3R,4S)-1-(4-fluorophenyl)-2-oxo-4-[4-(trityloxy)phenyl]azetidin-3-yl}propionate (Vh; Z = CO₂Me)

A mixture of methyl 3-[(2S,3R)-1-(4-fluorophenyl)-2-(4-hydroxyphenyl)-4-oxoazetidin-3-yl]propionate (Va; Z = CO₂Me) (5 g, 20.5 mmol), triethylamine (3 ml), triphenylchloromethane (6.6 g, 23.7 mmol) in acetone (19 ml) was stirred at room temperature for 3 h. Water (2.6 ml) was added to a suspension, cooled to 15 °C and filtered. The precipitate was washed with 50% aq. acetone (1.9 ml) and water (4.3 ml). The solid was suspended in 50% aq. acetone (18 ml) and stirred at 15 - 18 °C for 1 h. The precipitate (10.63 g) was filtered, washed with water and dried in a vacuum oven at 45 °C over P₂O₅ for 10 h. Crude product was purified by silica gel chromatography with hexane/ethyl acetate (10/1) to give pure ester (Vh; Z = CO₂Me) (6.64 g, 55%) as a white solid with mp 138-140 °C.

### Example 24. Methyl 3-{(2S,3R)-2-[4-(tert-butyldimethylsilyloxy)phenyl]-1-(4-fluorophenyl)-4-oxoazetidin-3-yl}propionate (Vi; Z = CO₂Me)

A mixture of methyl 3-[(2S,3R)-1-(4-fluorophenyl)-2-(4-hydroxyphenyl)-4-oxoazetidinyl]propionate (Va; Z = CO₂Me) (500 mg, 1.46 mmol), tert-butyldimethylsilyl chloride (850 mg, 3.64 mmol) and imidazole (11 mg, 0.03 mmol) in N,N-dimethylformamide (10 ml) was stirred at 35 °C for cca. 5 h. The mixture was cooled to room temperature, then 5% solution of sodium hydrogencarbonate (10 ml) and diethyl ether were added. The organic layer was washed with water, dried over sodium sulfate and concentrated in vacuo. The residue was purified by silica gel chromatography with toluene/ethyl acetate (9/1) to give pure ester (Vi; Z = CO₂Me) (541 mg, 71%) as a brown colored oil.

### Example 25. Methyl 3-{(3R,4S)-1-(4-fluorophenyl)-2-oxo-4-[4-(tetrahydro-2H-pyran-2-yloxy)phenyl]azetidin-3-yl}propanoate (Vk; Z = CO₂Me)

A mixture of methyl 3-[(2S,3R)-1-(4-fluorophenyl)-2-(4-hydroxyphenyl)-4-oxoazetidinyl]propionate (Va; Z = CO₂Me) (1.0 g, 2.91 mmol), pyridinium toluene-4-sulfonate (0.92 g, 2.94 mmol) and 3,4-dihydro-2H-pyran (0.43 g, 5.6 mmol) in methylene chloride (40 ml) was stirred at room temperature for 17h. Then 5% solution of sodium hydrogencarbonate (10 ml) and diethyl ether (40 ml) were added, the organic phase was washed with water, dried over sodium sulfate and concentrated in vacuo. Ester (Vk; Z = CO₂Me) was obtained (0.81 g) as an almost colorless oil.

### Example 26. Crystallization of ezetimibe (anhydro form A)

1 g of ezetimibe (anhydro form A) was dissolved in a selected solvent by heating at reflux. The choice and volume of the solvent is shown in Table 1. The resulting solution was allowed to cool to room temperature with magnetic stirring or further down to 0 °C. The ultimate temperature of cooling (Tᵤ) is also indicated in Table 1. The solid was collected by filtration, suction dried for cca. 10 min, then dried in a desiccator at 20 °C (RH below 15%) for 16 h and analyzed. Precipitates obtained from propionitrile and α,α,α-trifluorotoluene were dried in a vacuum oven at 50 °C for 16 h. XRPD results are given in Table 1. All samples melted within the range of 156-164 °C, except for the sample obtained from tert-butanol (form S), which melted partially at 83-86 °C, then resolidified and melted again at 156-160 °C.

**Table 1.**

| Solvent | Vol. | Tᵤ | Yield | Resulting cryst.form |
|---|---|---|---|---|
| | (ml) | (°C) | (%) | |
| Isopropyl acetate | 3 | 20 | 61.5 | A |
| Butyl acetate | 3 | 0 | 56 | A |
| Nitromethane | 3 | 20 | 90 | A |
| Acetonitrile | 3 | 20 | 69 | A |
| Propionitrile | 3 | 0 | 34 | A |
| Toluene | 17 | 20 | 89 | A |
| Chlorobenzene | 6 | 20 | 76.5 | A |
| α,α,α-Trifluorotoluene | 167 | 0 | 57 | A |
| Anisol | 1.9 | 20 | 92.5 | A |
| Cyclopentyl methyl ether | 1.9 | 20 | 90 | A |
| 2-Methyltetrahydrofuran | 1 | 20 | 85 | A |
| tert-Butyl methyl ether + | 22 | 20 | 92 | A |
| n-heptane | 25 | | | |
| tert-Butanol | 1.9 | 20 | 86 | S |
| Tetrachloroethylene | 75 | 20 | 90 | A |
| Acetic acid/water (6/1 v/v) | 3.5 | 20 | 78 | A/H ≈ 1/9 |
| Methanol + water | 2.5 0.5 | 0 | 80.5 | H |

### Example 27. Crystallization of ezetimibe (hydrated form H)

1 g of ezetimibe (hydrated form H) was dissolved in a selected solvent by heating at reflux. The choice and volume of the solvent is shown in Table 2. The resulting solution was allowed to cool to room temperature with magnetic stirring. The solid was collected by filtration, suction dried for cca. 10 min, then air-dried at 20 °C (RH of 35-45%) till constant weight and analyzed. XRPD results are given in Table 2. All samples melted within the range of 156-164 °C, except for the sample obtained from tert-butanol (form S), which melted first at 87-94 °C, recrystallized at 96 °C and melted second at 159-162 °C.

**Table 2.**

| Solvent | Vol. | Yield | Resulting cryst.form |
|---|---|---|---|
| | (ml) | (%) | |
| Isopropyl acetate | 2.3 | 87 | H + A |
| Butyl acetate | 1.5 | 88.5 | H > A |
| Nitromethane | 1.4 | 94 | H >> A |
| Acetonitrile | 1.5 | 82.5 | H > A |
| Propionitrile | 1.4 | 60 | H >> A |
| Toluene | 16 | 92 | H + A |
| Chlorobenzene | 5.7 | 92.5 | H + A |
| Anisol | 2 | 76 | H + A |
| Cyclopentyl methyl ether | 2.4 | 81.5 | H + A |
| 2-Methyltetrahydrofuran | 1 | 53.5 | H + A |
| tert-Butyl methyl ether + | 22 | 99 | A |
| n-heptane | 25.5 | | |
| tert-Butanol | 1.9 | 89.5 | S |
| Acetic acid | 1.4 | 95.5 | H + A |

| | | | |
|---|---|---|---|
| Explanation of symbols: H >> A: form A is present in traces; H > A: form A present in small amount; H + A: both forms present in substantial amounts by XRPD | | | |

### Example 28. Crystallization of ezetimibe (anhydro form A) from ethanol.

1 g of ezetimibe (anhydro form A) was dissolved in ethanol by heating at reflux. The grade and volume of the solvent is shown in Table 3. The resulting solution was magnetically stirred at room temperature for 1 h and at 0 °C for 2 h. The solid was collected by filtration, suction dried for cca. 10 min, then air-dried at 21 °C and 36% RH for 16 h, and analyzed by XRPD. Results are given in Table 3. Both samples melted within the range of 157-164 °C.

**Table 3.**

| Solvent | Vol. | Yield | Resulting cryst.form |
|---|---|---|---|
| | (ml) | (%) | |
| 96% Ethanol | 2 | 43 | A<H |
| Abs. ethanol | 2 | 42 | A>H |

### Example 29. Crystallization of ezetimibe by slow concentration of ethanol solution.

0.50 g of ezetimibe (anhydro form A) was dissolved in 1 ml of warm ethanol. The clear solution was concentrated on a rotary evaporator at cca. 250 mbar starting pressure, which was gradually decreased to the ultimate pressure of cca. 50 mbar. The grade of ethanol and heating bath temperature are given in Table 4. Viscous oily residue formed initially, which solidified soon. When constant weight was achieved, the samples were analyzed directly by XRPD. Results are given in Table 4. All samples melted within the range of 158-162.5 °C.

**Table 4.**

| Ethanol | Bath T (°C) | Resulting cryst.form |
|---|---|---|
| Abs. | 44 | A |
| Techn. grade | 44 | A + H |
| Abs. | 23 | A |
| Techn. grade | 23 | A + H |
| 96% | 23 | A + H |

### Example 30. Crystallization of ezetimibe (anhydro form A) from aqueous methanol.

27.0 g of ezetimibe (anhydro form A) was dissolved in a mixture of methanol (120 ml) and water (24 ml) by heating at reflux temperature. The resulting solution was allowed to cool to room temperature, then cooled in an ice bath for 30 min. The solid was collected by filtration, washed with an ice-cold methanol/water (2/1) mixture (54 ml) and air-dried at 20 °C and cca. 40% RH for 16 h. Hydrated form of ezetimibe H (25.78 g, 91.5%) with mp. 158-161 °C was obtained, which contained 4.5% water according to KF analysis. LOD experiments are given in Example 37.

### Example 31. Crystallization of ezetimibe (hydrated form H) from aqueous tert-butanol.

1.04 g of ezetimibe (hydrated form H) was dissolved in tert-butanol/water mixture (10/1, 5 ml) by heating at reflux temperature. The resulting solution was cooled to room temperature and stirred mechanically until thickening took place (cca. 1 h). The solid was collected by filtration and air-dried overnight. Hydrated form of ezetimibe H (0.77 g, 74.5%) with mp. 160-162 °C was obtained according to XRPD analysis, which contained 6.2% water by KF analysis and showed LOD of -5.5% at 130 °C.

### Preparation of ezetimibe tert-butanol solvate (S)

### Example 32. Crystallization of ezetimibe (anhydro form A) from tert-butanol.

### Procedure 1.

5.06 g of ezetimibe (anhydro form A) was dissolved in tert-butanol (9.5 ml) by heating at reflux temperature. While stirred magnetically, the resulting solution was allowed to cool to room temperature. The solid was collected by filtration and dried in desiccator for 16 h. Pure S form of ezetimibe (tert-butanol solvate) (5.43 g) was obtained according to XRPD analysis, which melted first at 86-90 °C, resolidified above 96 °C and melted second at 155-160 °C. Sample was 99.2% pure by HPLC, contained 1.5% of water according to KF analysis, and showed LOD of -11.5% at 130 °C. ¹H-NMR (DMSO-d₆): δ = 1.11 (s, 6.0 H, t-Bu), 1.6-1.9 (m, 4 H, H-1', H-2'), 3.08 (m, 1 H, H-3), 4.20 (s, 0.7 H, t-Bu-OH), 4.49 (m, 1 H, H-3'), 4.80 (d, J = 2.3 Hz, 1 H, H-4), 5.29 (br d, J = 2.7 Hz, 1 H, OH-3'), 6.73-6.78 (m, 2 H, Ar-H), 7.08-7.34 (m, 10 H, Ar-H), 9.54 (br s, 1 H, Ar-OH). Both NMR and KF analyses showed the structure of solvate to be ezetimibe · 0.67 tert-BuOH · 0.33 H₂O in this particular case.

### Procedure 2.

5.13 g of ezetimibe (anhydro form A) was dissolved in tert-butanol (12 ml) by heating at reflux temperature. While stirred mechanically, the resulting solution was allowed to cool to room temperature. The solid was collected by filtration and dried in desiccator for 16 h. Pure S form of ezetimibe (tert-butanol solvate) (5.64 g) was obtained according to XRPD analysis, which melted first at 86-90 °C, resolidified above this temperature and melted second at 158-161 °C. Sample was 99.4% pure by HPLC, contained 0.67% of water according to KF analysis and showed LOD of-12.0% at 130 °C within 4.5 min.

### Example 33. Crystallization of ezetimibe (hydrated form H) from tert-butanol.

Procedure 1. 5.01 g of ezetimibe (hydrated form H) was dissolved in tert-butanol (9 ml) by heating at reflux temperature. While stirred magnetically, the resulting solution was allowed to cool to room temperature. The solid was collected by filtration and air-dried for 3 d. Pure S form of ezetimibe (tert-butanol solvate) (5.34 g) was obtained according to XRPD analysis, which melted first at 87-90 °C, resolidified above this temperature and melted second at 161-163 °C. Sample was 99.8% pure by HPLC, contained 1.1 % of water according to KF analysis, and showed LOD of -10.3% at 130 °C.

Procedure 2. 5.04 g of ezetimibe (hydrated form H) was dissolved in tert-butanol (9 ml) by heating at reflux temperature. While stirred mechanically, the resulting solution was allowed to cool to room temperature. No precipitation took place in this case even after 3 days, but after seeding with crystals of ezetimibe tert-butanol solvate crystallization did occur. The solid was collected by filtration and air-dried overnight. Pure S form of ezetimibe (tert-butanol solvate) (5.43 g) was obtained according to XRPD analysis, which melted first at 84-89 °C, resolidified above this temperature and melted second at 162-163.5 °C. Sample was 99.6% pure by HPLC, contained 0.95% of water according to KF analysis, and showed LOD of - 13.1 % at 130 °C.

### Example 34. Slurrying of ezetimibe (anhydro form A) in tert-butanol.

Procedure 1. 1.0 g of ezetimibe (anhydro form A) was slurried in tert-butanol (2.5 ml) at room temperature. While stirred magnetically the mixture thickened considerably within 2 h. The solid was collected by filtration and dried in desiccator for 3 d. Ezetimibe tert-butanol solvate (1.05 g) was obtained in admixture with a trace of anhydro form (S >> A) according to XRPD analysis, which melted first at 81-83 °C, resolidified above this temperature and melted second at 152-155.5 °C. The sample contained 0.48% of water according to KF analysis and showed LOD of -10.5% at 130 °C.

Procedure 2. 1.01 g of ezetimibe (anhydro form A) was slurried in tert-butanol (2.5 ml) at room temperature. While stirred mechanically the mixture thickened considerably within 7 h. The solid was collected by filtration and dried in desiccator for 3 d. Ezetimibe tert-butanol solvate (1.05 g) was obtained in admixture with a trace of anhydro form (S >> A) according to XRPD analysis, which melted partially at 87-90 °C, resolidified above this temperature and melted second at 156-160 °C. The sample contained 0.48% of water according to KF analysis and showed LOD of -11.8% at 130 °C.

### Example 35. Slurrying of ezetimibe (hydrated form H) in tert-butanol.

### Procedure 1.

2.01 g of ezetimibe (hydrated form H) was slurried in tert-butanol (5 ml) at room temperature. While stirred magnetically the mixture thickened considerably within 10 min. The solid was collected by filtration and air-dried for 16 h. Ezetimibe tert-butanol solvate (2.10 g) was obtained in admixture with a trace amount of hydrated form (S >> H) according to XRPD analysis, which melted first at 85.5-90.5 °C, resolidified above 106 °C and melted second at 156-160 °C. The sample contained 0.51% of water according to KF analysis and showed LOD of -12.8% at 130 °C.

### Procedure 2.

2.03 g of ezetimibe (hydrated form H) was slurried in tert-butanol (5 ml) at room temperature. While stirred mechanically the mixture thickened considerably within 45 min. The solid was collected by filtration and air-dried for 20 h. Ezetimibe tert-butanol solvate (2.17 g) was obtained in admixture with a trace amount of hydrated form (S >> H) according to XRPD analysis, which melted first at 87-92 °C, resolidified above this temperature and melted second at 160-163 °C. The sample contained 0.48% of water according to KF analysis and showed LOD of-12.4% at 130 °C.

### Drying of the hydrated form H of ezetimibe

### Example 36. Water sorption/desorption properties of ezetimibe.

Anhydro form of ezetimibe was tested on the automatic water sorption analyzer DVS-1 (Surface Measurement Systems Ltd., London, GB) under the following conditions:
- controlled room temperature (25 °C)
- nitrogen flow 200 ml/min
- two full cycles from 0% RH to 95% RH and back in eleven stages
- minimal time per one stage (when dm/dt < 0.002%) was 10 min
- maximal time per one stage was 360 min.

Results: In the first cycle, no significant absorption of water was observed up to 50% RH. At 60% RH the water sorption affinity became very high and reached equilibrium at 4.2% mass change. At even higher relative humidities the water sorption was only slightly increased (4.4% total at 95% RH). In the desorption cycle the mass was not significantly changed down to 30% RH while it dropped sharply at 20% RH. The second cycle took a similar course except that the water sorption increased sharply already at 50% RH.

### Example 37

Hydrated form H of ezetimibe (from Example 30) was heated in a Mettler HR73 Halogen Moisture Analyzer. Constant value of LOD of -4.5% was achieved after 11.5 min at 50 °C, 6.5 min at 60 °C, or 4.5 min at 70 °C. In all cases anhydro form of ezetimibe resulted according to XRPD analysis.

### Example 38

Hydrated form H of ezetimibe was dried in an air dryer (RH was 35%) at two different temperatures. Drying was very fast at 40 °C, when only anhydro form of ezetimibe was detected after 1 h according to XRPD analysis.

### Example 39

Hydrated form H of ezetimibe was dried in a vacuum dryer at an ambient temperature and a pressure of cca. 100 mbar. After 1 h anhydrous form already predominated (A > H), after 2 h it became the sole form according to XRPD analysis.

### Drying of the ezetimibe tert-butanol solvate

### Example 49

Pure S form of ezetimibe (tert-butanol solvate; from Example 32) was heated in a Mettler HR73 Halogen Moisture Analyzer at different temperatures for the time indicated and analyzed by XRPD. Results are shown in Table 5.

**Table 5.**

| Temperature | LOD (%) | Time (min) | Resulting |
|---|---|---|---|
| (°C) | | | cryst.form |
| 50 | -0.83 | 14 | S |
| 60 | -1.10 | 10.5 | S |
| 70 | -2.34 | 9 | S |
| 130 | -12.0 | 4.5 | A |

### Examples 41-60

The compositions for examples 1-19 are given in table 7:

| **Example No.** | **1** (mg) | **2** (mg) | **3** (mg) | **4** (mg) | **5** (mg) | **6** (mg) | **7** (mg) | **8** (mg) | **9** (mg) | **10** (mg) | **11** (mg) | **12** (mg) | **13** (mg) | **14** (mg) | **15** (mg) | **16** (mg) | **17** (mg) | **18** (mg) | **19** (mg) |
|---|---|---|---|---|---|---|---|---|---|---|---|---|---|---|---|---|---|---|---|
| **Ingredient** | | | | | | | | | | | | | | | | | | | |
| Ezetimibe | 10 | 10 | 10 | 10 | 10 | 10 | 10 | 10 | 10 | 10 | 10 | 10 | 10 | 10 | 10 | 10 | 10 | 10 | 10 |
| Lactose monohydrate | 65 | - | - | 53 | 47 | 49 | - | - | - | 57.8 | 53 | - | - | 47.5 | 51 | 49 | - | 55 | 55 |
| Mannitol | - | - | 52.8 | - | - | - | 54 | - | 50 | - | - | - | 41 | - | - | - | 49 | - | - |
| Calcium phosphate | - | 55 | - | - | - | - | - | 40 | - | - | - | 52 | - | - | - | - | - | - | - |
| Microcrystalline cellulose | 20 | 20 | 25 | 25 | 20 | 30 | 25 | 35 | 30 | 20 | 20 | 25 | 30 | 30 | 30 | 30 | 29 | 20 | 24 |
| Povidone K25-K30 | 1 | 4 | 3 | 3 | 3 | - | - | - | - | - | - | - | - | - | 2 | 2 | | 4 | - |
| HPC Klucel EF^{®} | - | - | - | - | - | 2 | 4 | 4 | 3 | - | - | - | - | - | - | - | 3 | - | - |
| PMC Pharmacoat^{®}603 | - | - | - | - | - | - | - | - | - | 2 | 3 | 5 | 4 | 3 | - | - | - | - | 2 |
| Croscarmellose Na (Ac-di-sol) | 1 | 8 | - | - | 6 | 6 | 4 | - | - | 7 | - | 5 | - | 6 | 4 | 6 | 8 | 8 | 6 |
| Sodium starch glycolate | - | - | 6 | - | - | - | - | 8 | - | - | 10 | - | - | - | - | - | - | - | - |
| Crospovidone | - | - | - | 5 | - | - | - | - | 4 | - | - | - | - | - | - | - | - | - | - |
| L-HPC LH-21 | - | - | - | - | 10 | - | - | - | - | - | - | - | 12 | - | - | - | 8 | - | - |
| Sodium laurilsulfate | 2 | 2 | 2 | 2 | 2 | - | - | 2 | 2 | 2 | 2 | - | 2 | - | - | 2 | 2 | 2 | 2 |
| Polysorbate 80 | - | - | - | - | - | 2 | 2 | - | - | - | - | 2 | - | 2 | 2 | - | - | - | - |
| Talc | - | - | - | 1 | 1 | - | - | - | - | - | 1 | - | - | - | - | - | - | - | |
| Silica | - | - | 0.2 | - | - | - | - | - | - | 0.2 | - | - | - | 0.2 | - | - | - | - | - |
| Magnesium stearate | - | 1 | - | 1 | 1 | - | 1 | 1 | - | 1 | - | 1 | 1 | - | - | - | 1 | 1 | - |
| Sodium stearyl fumarete | 1 | - | 1 | - | - | 1 | - | - | 1 | - | 1 | - | - | 1.3 | 1 | 1 | - | - | 1 |
| TOTAL | 100 | 100 | 100 | 100 | 100 | 100 | 100 | 100 | 100 | 100 | 100 | 100 | 100 | 100 | 100 | 100 | 100 | 100 | 100 |

For the preparation of pharmaceutical compositions ezetimibe, filler (lactose monohydrate, mannitol or calcium phosphate) and disintegrant (Ac-di-sol, Primojel, L-HPC or crospovidone) are mixed. Binder (povidone, HPC or HPMC) is dissolved in purified water, solubilizing agent (sodium lauril sulfate or polysorbate 80) is added and the obtained solution is sprayed onto the powder mixture in fluid bed granulator. Alternatively, ezetimibe is suspended in granulation mixture and sprayed onto powder mixture.

Glidant (talc or silica, colloidal anhydrous) and lubricant are admixed and the obtained mixture is pressed into tablets using appropriate compression tool. Alternatively, only part of disintegrant is added intragranularly and the rest added to granules.

## Claims

1. A process for preparing a compound represented by general formula 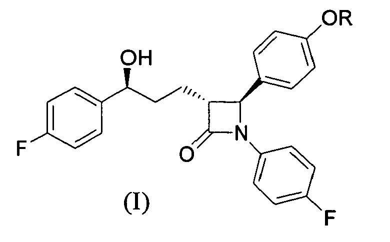 wherein
R represents a hydrogen atom, a protective group selected from the group consisting of trisubstituted silyl, arylmethyl, mono or disubstituted arylmethyl with the substituents, elected from the group consisting of halides, methoxy, nitro, phenyl, naphthyl and any combinations thereof,
comprising the steps of
a) metal-catalysed asymmetric transfer hydrogenation of p-fluoroacetophenones of general formula (II) 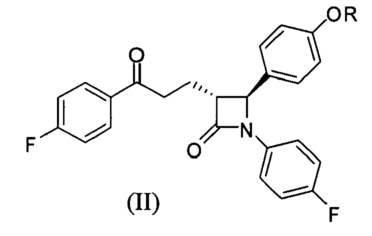 wherein R has the same meaning as above
by using of a hydrogen donor in the presence of a metal catalyst based on Ruthenium complexes
b) obtaining the compound represented by general formula (I)
c) optionally purifying the compound represented by general formula (I).

2. The process of claim 1, wherein R is selected from the group consisting of t-butyldimethylsilyl, t-butyldiphenylsilyl, triisopropylsilyl, trityl, benzyl, p-bromobenzyl, p-chlorobenzyl, p-nitrobenzyl, o-nitrobenzyl, p-phenylbenzyl, p-methoxybenzyl, **characterised in that** said process provides the compound represented by general formula (I) with a diastereometric ratio of more than 99:1.

3. The process of claim 1, wherein R is selected from the group consisting of p-bromobenzyl, p-chlorobenzyl, p-nitrobenzyl, p-methoxybenzyl, trityl, tert-butyldimethylsilyl and benzyl.

4. The process of any of the proceeding claims, wherein the metal catalyst is based on Ruthenium complex of optically active N-sulfamoyl-1,2-diamine ligands of the general formula (VI): 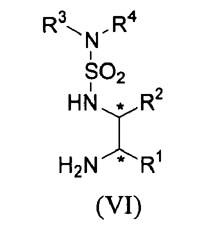 wherein:
- C* represents an asymmetric carbon atom;
- R¹ and R² independently represent a hydrogen atom, an optionally substituted aryl, or cycloalkyl, or R¹ and R² may be linked together to form a cyclohexane ring;
- R³ and R⁴ independently represent a hydrogen atom, a C₁₋₁₅ alkyl, linear or branched, optionally substituted with an aryl; or R³ and R⁴ may be linked together to form with the nitrogen atom an optionally substituted C₄₋₆ ring.

5. The process of claim 4 wherein the optically active N-sulfamoyl-1,2-diamine ligands has enantiomer excess more than 99%.

6. The process of claim 4 wherein R³ and/or R⁴ can be selected from the group consisting of methyl, iso-propyl and cyclohexyl.

7. The process of claim 4 wherein R³ and R⁴ are linked together to form a ring selected from the group consisting of pyrrolidyl, piperidyl, morpholyl and azepanyl.

8. The process of any of the proceeding claims wherein the metal catalyst is prepared from a ruthenium metal precursor and an optically active N-sulfamoyl-1,2-diamine ligand of the general formula (VI).

9. The process of claim 8 wherein the ruthenium catalyst precursor consists of η⁶-arene-ruthenium(II) halide dimers of the formula [RuX₂(η⁶-arene)]₂, wherein η⁶-arene represents an arene, selected from the group consisting of benzene, p-cymene, mesitylene, 1,3,5-triethylbenzene, hexamethylbenzene and anisole, and X is halide selected from the group consisting of chloride, bromide and iodide.

10. The process of any of the proceeding claims wherein the hydrogen donor is based on HCO₂H.

11. The process of claim 10 wherein the hydrogen donor is selected from the group consisting of HCO₂H-Et₃N, HCO₂H-iso-Pr₂NEt, HCO₂H-metal bicarbonates and HCO₂H-metal carbonates wherein the metal is selected from the group consisting of Na, K, Cs, Mg and Ca.

12. The process of any of the proceeding claims wherein the metal-catalysed asymmetric transfer hydrogenation is conducted in solvent selected from the group consisting of dichloroethane, acetonitrile, N,N-dimethyl formamide, N,N-dimethylacetamide, 1-methyl-2-pyrrolidinone (NMP), 1,1,3,3-tetramethylurea, 1,3-dimethyl-2-imidazolidinone, N,N'-dimethylpropyleneurea and mixtures thereof.

13. The process of any of the proceeding claims, wherein the compound of formula (I) is ezetimibe.

14. Ezetimibe form S specified by an X-ray powder diffraction pattern exhibiting peaks at the following diffraction angles: 7.3, 15.3, 16.7, 18.7, 21.8, 24.0°2Th.

15. Ezetimibe form S of claim 14 specified by an X-ray powder diffraction pattern exhibiting additional peaks at the following diffraction angles: 6.2, 20, 25.3°2Th.

16. Ezetimibe form S of claim 14 and 15 having an X-ray powder diffraction pattern as shown in Figure 3.

17. Ezetimibe form S specified by ¹H-NMR peaks at δ = 1.11 (s, t-Bu), 1.6-1.9 (m, 4 H, H-1', H-2'), 3.08 (m, 1 H, H-3), 4.20 (s, t-Bu-OH), 4.49 (m, 1 H, H-3'), 4.80 (d, J = 2.3 Hz, 1H, H-4), 5.29 (br d, J = 2.7 Hz, 1 H, OH-3'), 6.73-6.78 (m, 2 H, Ar-H), 7.08-7.34 (m, 10 H, Ar-H), 9.54 (br s, 1 H, Ar-OH).

18. Ezetimibe form S of claims 14 to 17 **characterized by** a water content of about 0 to about 2%, determined by Karl Fischer analysis.

19. Ezetimibe form S of claim 18 **characterized by** a water content of about 0.5 to about 1.5% determined by Karl Fischer analysis.

20. Ezetimibe form S of claims 14 to 17 wherein the purity is more than 90%, preferably more than 99%, more preferably more than 99.6%.

21. Ezetimibe form S of claims 14 to 17 wherein said ezetimibe form S contains from about 8 to about 15% of tert-butanol, preferably from about 10 to about 12% of tert-butanol.

22. Ezetimibe form S of claims 14 to 17 wherein the crystals of said ezetimibe form S have a particle size of less than about 100 microns, preferably less than 50 microns, more preferably less than about 30 microns.

23. Ezetimibe form S of claims 14 to 17 wherein the micronized crystals of said ezetimibe form S have a particle size of less than about 30 microns, preferably less than 20 microns, more preferably less than about 10 microns.

24. Ezetimibe form S of claims 14 to 17 wherein said ezetimibe form S contains not more than 20%, preferably not more than 10%, more preferably not more than 5% and most preferably not more than 1% of other polymorphic forms.

25. The process for the preparation of ezetimibe form S of claims 14 to 24 comprising the steps of:
a) dissolving ezetimibe of any polymorphic form in tert-butanol
b) cooling the resulting solution to room temperature
c) drying.

26. A pharmaceutical composition comprising a therapeutically effective amount of ezetimibe prepared according to any of the proceeding claims optionally mixed with one or more active substances, and one or more pharmaceutically acceptable ingredients.

27. A pharmaceutical composition comprising a therapeutically effective amount of ezetimibe form S optionally mixed with one or more active substances, and one or more pharmaceutically acceptable ingredients.

28. The use of a therapeutically effective amount of ezetimibe prepared according to any of the proceeding claims for lowering cholesterol levels in mammal in need of such treatment.

29. The use of a therapeutically effective amount of ezetimibe form S for lowering cholesterol levels in mammal in need of such treatment.

30. A compound of the formula

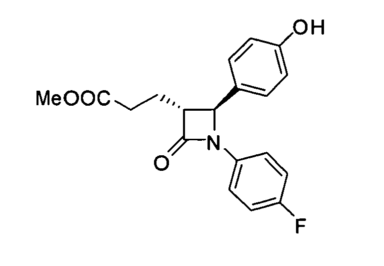

31. A compound of the formula

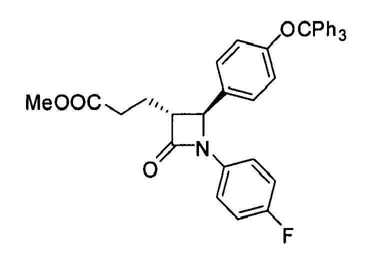

32. A compound of the formula
